# EUROPEAN PATENT APPLICATION

(11) **EP 4 310 096 A1**
(43) Date of publication of application: **24.01.2024**
(21) Application number: 22771420.1
(22) Date of filing: 15.03.2022
(51) Int. Cl.: C07K 14/47, C07K 16/00, C12N 15/12

(54) **COMPLEX OR SALT THEREOF, AND METHOD FOR MANUFACTURING SAME**

(30) Priority: 16.03.2021 JP 2021042918
(71) Applicant: AJINOMOTO CO., INC., Chuo-ku Tokyo 104-8315 (JP)
(72) Inventor: YAMADA, Kei, Kawasaki-shi, Kanagawa 210-8681 (JP); MATSUDA, Yutaka, Kawasaki-shi, Kanagawa 210-8681 (JP); INOUE, Ippei, Kawasaki-shi, Kanagawa 210-8681 (JP); SHIKIDA, Natsuki, Kawasaki-shi, Kanagawa 210-8681 (JP); SHIMBO, Kazutaka, Kawasaki-shi, Kanagawa 210-8681 (JP)
(74) Representative: Mewburn Ellis LLP
(86) International application number: PCT/JP2022/011534
(87) International publication number: WO 2022/196675

(57) **Abstract**

The present invention provides a highly controlled complex comprising an antibody and ferritin particles. More specifically, the present invention provides the inventions of the following (I) and (II).
(I) A complex or salt thereof comprising (A) one IgG antibody and (B) two human ferritin particles comprising one or more human ferritin H chains linked thereto.
(II) A method for producing a complex or salt thereof, comprises the following (1)-(3):
(1) introducing first reactive groups into an IgG antibody to form a modified IgG antibody comprising two first reactive groups;
(2) introducing a second reactive group capable of reacting with the first reactive group into human ferritin particles comprising one or more human ferritin H chains to form modified particles comprising one or more modified human ferritin H chains comprising the second reactive group; and
(3) reacting the modified IgG antibody with the modified particles to obtain the complex.

## Description

### Field

The present invention relates to a complex or salt thereof, and a method for producing the same.

### Background

A ferritin particle is a spherical protein that is ubiquitously present in organisms from animals and plants to microorganism and has an internal cavity formed by a plurality of monomers. In animals such as humans, it is known that two kinds of monomers of H and L chains are present as monomers constituting the ferritin particle, and that the ferritin particle is a multimer composed of 24 monomers (in many cases, a mixture of H chain monomers and L chain monomers) having a caged-like configuration with an outer diameter of 12 nm and an internal cavity with an inner diameter of 7 nm. In addition, the N-terminus of the monomer constituting the ferritin particle is exposed on the surface of the 24-mer, but the C-terminus of the monomer is not exposed on the surface and is present in the internal cavity. The ferritin particle can retain iron in its internal cavity and is deeply involved in the homeostasis of iron elements in living bodies or cells by playing the role of physiological functions such as transport and storage of iron. The ferritin particle may also be relatively easily dissociated and associated, such that they are in the form of a 24-mer or monomer or in the co-present form thereof depending on the conditions such as the solvent comprising it. Thus, by suitably adjusting the conditions such as the solvent comprising the ferritin particle, the 24-mer can be dissociated into monomers, and the dissociated monomers can be reassociated into the 24-mer. It is also known that when a given substance was allowd to be co-exist with the ferritin monomer in the solvent upon reassociation to encapsulate the substance in the internal cavity of the reassociated 24-mer.

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) which is jointly developed by Immunogen and Roche.

ADCs including T-DM1 have had the problem of their nonuniformity from the beginning of their development. That is, a drug antibody ratio (DAR) and a conjugation position are not constant. It is known that such a random conjugation method normally provides a DAR within a range of 0 to 8, producing a plurality of antibody medicines having different numbers of bonds of a drug. In recent years, it has been reported that when the number of bonds and the bond positions of a drug(s) to an ADC are changed, pharmacokinetics, and a releasing rate and effects of the drug change. Given these circumstances, next-generation ADCs are required to control the number and positions of a drug to be conjugated. It is believed that when the number and positions are fixed, the problems of expected efficacy, variations in conjugation medicines, and lot difference, or what is called regulation, will be solved.

For the ferritin particle, complexes with an antibody are also known.

Patent Literature 1 reports a complex of an antibodies (nanobodies) and a ferritin particle, which comprises fusion proteins of the antibody (nanobodies) and the ferritin monomer as a monomeric unit. Because this complex uses the fusion protein of the antibody and the ferritin monomer, the ratio of the antibody and the ferritin particle (antibody/ferritin particle) corresponds to the number of ferritin monomers that constitute the ferritin particle. Thus, if the ferritin particle is a homo 24-mer of the fusion protein of the antibody (nanobody) and the ferritin monomer, the number of antibodies per ferritin particle is 24.

Non-Patent Literature 1 describes a complex of IgG antibodies and a ferritin particle linked using glutaraldehyde. In the method described in Non-Patent Literature 1, antibodies are introduced randomly into a ferritin particle using glutaraldehyde. Therefore, regioselectivity is not achieved in the linking sites both of the ferritin particle to the antibodies (i.e. sites in the ferritin monomer used for linking with the antibodies) and of the antibodies to the ferritin particle. In addition, the method described in Non-Patent Literature 1 cannot control the number of antibodies per ferritin particle.

Non-Patent Literature 2 describes a complex of a monoclonal antibody or monoclonal antibodies and a ferritin particle linked by a covalent bond. In this complex, the ferritin particle is constructed using ferritin L chains into which mutations have been introduced by genetic engineering techniques. In addition, this complex is not considered to be regioselective at the linking sites of the ferritin particle to the antibodies. Furthermore, the method described in Non-Patent Literature 2 cannot strictly control the number of antibodies introduced into the ferritin particle to a specific value, and produces a complex with the number of antibodies per ferritin particle ranging from 1 to 4 (a mixture of multiple complexes comprising an antibody or antibodies and a ferritin particle at different ratios).

Non-Patent Literature 3 describes a complex of monoclonal antibodies and a ferritin particle linked by a covalent bond. In this complex, regioselectivity is not achieved at the linking site of the antibodies to the ferritin particle. In addition, the regioselectivity at the linking sites of the ferritin particle to the antibodies is unclear. Furthermore, the method described in Non-Patent Literature 3 introduces the antibodies into the ferritin particle and produces a complex in which the number of antibodies per ferritin particle is 3.

Any complexes of the antibody or antibodies and the ferritin particle described in the above prior art are prepared based on the technical concept of introducing a plurality of antibodies to ferritin particles, and the number of antibodies per ferritin particle (antibody/ferritin particle) is one or more values. The ferritin particle is a protein which is larger in size than an antibody, and which comprises 24 ferritin monomer units available for linking, and 24 ferritin monomers per ferritin particle can be utilized as modification sites. Thus, the complexes described in the above prior art is designed by a technical concept based on one ferritin particle that introduces a plurality of antibodies into one ferritin particle.

### Citation List

### Patent Literature

Patent Literature 1: Chinese Patent Application Publication No. 108976299

### Non-Patent Literature

Non-Patent Literature 1: Proc Natl Acad Sci USA (1974), 71(5), 2033-2037
Non-Patent Literature 2: Bioconj. Chem (2018), 29(4), 1209-1218
Non-Patent Literature 3: Nanoscale (2013), 5(24), 12278-85

### Summary

### Technical Problem

Because high quality control is required in clinical applications, it is desirable that a complex of an antibody and a ferritin particle be highly controlled. Therefore, an object of the present invention is to provide a highly controlled complex comprising an antibody and a ferritin particle.

### Solution to Problem

The inventors have conceived of preparing a complex of an antibody and ferritin particles based on one antibody, which introduces a plurality of ferritin particles into one antibody, rather than the above technical concept based on one ferritin particle which introduces a plurality of antibodies into one ferritin particle. As a result of intensive studies, the present inventors have found that by linking human ferritin particles comprising one or more human ferritin H chains and an Ig antibody in a certain manner, a complex or salt thereof can be prepared which comprises (A) one IgG antibody and (B) two human ferritin particles comprising one or more human ferritin H chains linked thereto. In such complexs, the number of antibodies per ferritin particle (antibody/ferritin particle) is 0.5, which is in contrast to the above prior art. As far as the present inventors know, while the prior art have reported a complex in which the number of antibodies per ferritin particle (antibody/ferritin particle) is plural, it does not describe or suggest a complex in which the number of ferritin particles per antibody (ferritin particle/antibody) is highly controlled to plural (particularly 2).

The present inventors have also found that a thiol group (-SH) in the side chain of cysteine residues of the human ferritin H chain can be utilized to regioselectively modify the sulfur atoms in the side chain of cysteine residues at positions 91 and/or 103 according to the reference position of the natural-type human ferritin H chain, and thus to easily and highly control the number and position of the modifying groups introduced onto the ferritin surface, and that the techniques can be utilized for easy and regioselective introduction of an IgG antibody to the sulfur atoms in the side chain of the cysteine residues at position 91 and/or 103 according to the reference position of the natural-type human ferritin H chain. As far as the present inventors know, the prior art does not teach or suggest the technical concept of controlling the position of modifying groups and antibody introduced into the ferritin surface.

The present inventors have further found that by utilizing an amino group (-NH₂) in the side chain of a specific lysine residue in an IgG antibody, human ferritin particles can be easily and regioselectively introduced to the nitrogen atom in the side chain of the specific lysine residue of the IgG antibody.

As described above, the present inventors have succeeded in providing highly controlled complexes comprising an antibody and ferritin particles to complete the present invention.

Accordingly, the present invention is as follows.
[1] A complex or salt thereof comprising (A) one IgG antibody and (B) two human ferritin particles comprising one or more human ferritin H chains linked thereto.
[2] The complex or salt thereof according to [1], wherein the human ferritin particle is a 24-mer of the human ferritin H chain.
[3] The complex or salt thereof according to [1] or [2], wherein the human ferritin H chain is a natural-type human ferritin H chain which may lack a methionine residue at position 1.
[4] The complex or salt thereof according to any of [1] to [3], wherein the complex is represented by the following formula (1):

   X1-L1-Y-L2-X2 (1)

   wherein
   X1 and X2 each indicate a human ferritin particle comprising one or more human ferritin H chains,
   Y is an IgG antibody,
   L1 and L2 each indicate a linker, and
   the two human ferritin particles indicated by X1 and X2 are linked to two linkers indicated by L1 and L2, respectively, via an atom in the side chain of amino acid residues present at the same position in the human ferritin H chain contained therein.
[5] The complex or salt thereof according to [4], wherein the linker does not contain a peptide moiety.
[6] The complex or salt thereof according to [4] or [5], wherein the atom in the side chain of the amino acid residue present at the same position in the human ferritin H chain is the sulfur atom in the side chain of cysteine residue.
[7] The complex or salt thereof according to [6], wherein the cysteine residue is present at position 91 and/or position 103 according to the reference position of the natural-type human ferritin H chain.
[8] The complex or salt thereof according to any of [1] to [7], wherein the antibody is a chimeric monoclonal antibody, a humanized monoclonal antibody, or a human monoclonal antibody.
[9] A complex or salt thereof according to any of [4] to [8], wherein the IgG antibody indicated by Y is linked to two linkers indicated by L1 and L2 via an atom in the side chain of amino acid residues present at the same position in the two heavy chain constant regions.
[10] The complex or salt thereof according to [9], wherein the atom in the side chain of the amino acid residues present at the same position in the two heavy chain constant regions is the nitrogen atom in the side chain of lysine residue.
[11] The complex or salt thereof according to [10], wherein the lysine residue is present at position 246/248, 288/290, or 317 of the IgG antibody heavy chain according to EU numbering.
[12] The complex or salt thereof according to any of [1] to [11], wherein the human ferritin particles comprising one or more human ferritin H chains comprise one or more modified human ferritin H chains and one or more unmodified human ferritin H chains.
[13] The complex or salt thereof according to any of [1] to [12], wherein the human ferritin particle is a particle comprising, per particle, each human ferritin H chain of the following (a) to (c) in the following average number:
   (a) the number of the modified human ferritin H chains linked to the linker is 1;
   (b) the number of the modified human ferritin H chains which are not linked to the linker and which are introduced with a modifying group is n; and
   (c) the number of the unmodified human ferritin H chains is m;
      where n is a number from 0 to 23, m is a number from 0 to 23, and the sum of n and m is 23.
[14] The complex or salt thereof according to any of [1] to [13], wherein the human ferritin particles are particles comprising one or more unmodified human ferritin H chains on average.
[15] The complex or salt thereof according to any of [1] to [14], wherein the human ferritin particles are particles comprising 12 or more unmodified human ferritin H chains on average.
[16] The complex or salt thereof according to any of [13] to [15], wherein the modifying group comprises a reactive group.
[17] The complex or salt thereof according to [16], wherein the reactive group is a bioorthogonal functional group to a protein.
[18] The complex or salt thereof according to [16], wherein the bioorthogonal functional group to the protein comprises one or more partial structures selected from the group consisting of a maleimide moiety, an azide moiety, a ketone moiety, an aldehyde moiety, a thiol moiety, an alkene moiety, an alkyne moiety, a halogen moiety, a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety, an isonitrile moiety, a sydnone moiety, and a selenium moiety.
[19] The complex or salt thereof according to any of [13] to [15], wherein the modifying group comprises a functional substance.
[20] The complex or salt thereof according to [19], wherein the functional substance comprises one or more moieties selected from the group consisting of a peptide, a protein, a nucleic acid, a small organic compound, a chelator, a sugar chain, a lipid, a high polymers, and a metal.
[21] The complex or salt thereof according to any of [1] to [20], wherein the human ferritin particles have a substance in an internal cavity.
[22] A method for producing a complex or salt thereof,
   wherein the complex comprises (A) one IgG antibody and (B) two human ferritin particles comprising one or more human ferritin H chains linked thereto,
   wherein the method comprises the following (1)-(3):
      (1) introducing first reactive groups into an IgG antibody to form a modified IgG antibody comprising two first reactive groups;
      (2) introducing a second reactive group capable of reacting with the first reactive group into human ferritin particles comprising one or more human ferritin H chains to form modified particles comprising one or more modified human ferritin H chains comprising the second reactive group; and
      (3) reacting the modified IgG antibody with the modified particles to obtain the complex.
[23] The method according to [22], wherein the complex is represented by the following formula (1):

   X1-L1-Y-L2-X2 (1)

   wherein
   X1 and X2 each indicate a human ferritin particle comprising one or more human ferritin H chains,
   Y is an IgG antibody,
   L1 and L2 each indicate a linker, and
   the two human ferritin particles indicated by X1 and X2 are linked to two linkers indicated by L1 and L2, respectively, via an atom in the side chain of amino acid residues present at the same position in the human ferritin H chain contained therein.
[24] The method according to [23], wherein the linker does not contain a peptide moiety.
[25] The method according to [23] or [24], wherein the atom in the side chain of the amino acid residue present at the same position in the human ferritin H chain is the sulfur atom in the side chain of cysteine residue.
[26] The method according to [25], wherein the cysteine residue is present at position 91 and/or 103 according to the reference position of a natural-type human ferritin H chain.
[27] A method for producing a complex or salt thereof,
   wherein the complex comprises (A) one IgG antibody and (B) two human ferritin particles comprising one or more modified human ferritin H chains and one or more unmodified human ferritin H chains linked thereto,
   wherein the method comprises the following (1)-(4):
      (1) introducing first reactive groups into an IgG antibody to form a modified IgG antibody comprising two first reactive groups;
      (2) introducing a second reactive group capable of reacting with the first reactive group into human ferritin particles comprising two or more human ferritin H chains to form first modified particles comprising two or more modified human ferritin H chains comprising the second reactive group;
      (3) replacing a portion of the two or more modified human ferritin H chains in the first modified particles with unmodified human ferritin H chains to form second modified particles comprising one or more modified human ferritin H chains and one or more unmodified human ferritin H chains; and
      (4) reacting the modified IgG antibody with the second modified particles to obtain the complex.
[28] A method for producing a complex or salt thereof,
   wherein the complex comprises (A) one IgG antibody and (B) two human ferritin particles comprising one or more modified human ferritin H chains and one or more unmodified human ferritin H chains linked thereto,
   wherein the method comprises the following (1)-(4):
      (1) introducing first reactive groups into an IgG antibody to form an IgG antibody comprising two first reactive groups;
      (2) introducing a second reactive group capable of reacting with the first reactive group into human ferritin particles comprising two or more human ferritin H chains to form first modified particles comprising two or more modified human ferritin H chains comprising the second reactive group;
      (3) reacting the modified IgG antibody with the first modified particles to form a complex or salt thereof comprising (A') one IgG antibody and (B') two human ferritin particles comprising two or more modified human ferritin H chains linked thereto; and
      (4) replacing a portion of the two or more modified human ferritin H chains in the complex or salt thereof comprising (A') one IgG antibody and (B') two human ferritin particles comprising two or more modified human ferritin H chains linked thereto with an unmodified human ferritin H chain to obtain the complex or salt thereof comprising (A) one IgG antibody and (B) two human ferritin particles comprising one or more modified human ferritin H chains and one or more unmodified human ferritin H chains linked thereto.
[29] The method according to [27] or [28], wherein the complex is represented by the following formula (1):

   X1-L1-Y-L2-X2 (1)

   wherein
   X1 and X2 each indicate a human ferritin particle comprising one or more modified human ferritin H chains and one or more unmodified human ferritin H chains,
   Y is an IgG antibody,
   L1 and L2 each indicate a linker, and
   the two human ferritin particles indicated by X1 and X2 are linked to two linkers indicated by L1 and L2, respectively, via an atom in the side chain of amino acid residues present at the same position in the human ferritin H chain contained therein.
[30] The method according to [29], wherein the linker does not contain a peptide moiety.
[31] The method according to [30], wherein the atom in the side chain of the amino acid residues present at the same position in the human ferritin H chain is the sulfur atom in the side chain of cysteine residue.
[32] The method according to [31], wherein the cysteine residue is present at position 91 and/or 103 according to the reference position of the natural-type human ferritin H chain.
[33] The method according to any of [22] to [32], wherein the first reactive group and the second reactive group are a bioorthogonal functional group to a protein.
[34] The method according to [33],
   wherein the bioorthogonal functional group to the protein comprises one or more partial structures selected from the group consisting of a maleimide moiety, an azide moiety, a ketone moiety, an aldehyde moiety, a thiol moiety, an alkene moiety, an alkyne moiety, a halogen moiety, a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety, an isonitrile moiety, a sydnone moiety, and a selenium moiety,
   wherein the first reactive group and the second reactive group are selected in a combination capable of reacting with each other.

### Effect of Invention

According to the present invention, the ratio of an antibody and ferritin particles can be highly controlled. Also, regioselectivity can be achieved at both the linking site of the ferritin particle to the antibody and the linking site of the antibody to the ferritin particle. Therefore, the present invention can achieve extremely high quality control.

In addition, human ferritin particles comprising natural-type human ferritin monomers and/or a linker that do not contain a peptide can be used to avoid immunogenicity upon clinical application to humans.

Furthermore, in the complexes or salts thereof of the present invention, the antibody and ferritin particles can each serve as a drug and/or targeting agent. This is because the antibody itselves can be used as the drug and/or targeting agent, and ferritin particles can also be used as a means for drug delivery and/or a targeting agent. For example, ferritin particles can be used as a means for drug delivery since they can encapsulate a large amount of substances in an internal cavity and they can bind a drug to a ferritin monomer on their surface. In particular, where the drug has properties that are prone to degradation in living bodies (e.g., nucleic acid molecules such as siRNA), ferritin is useful as a means for drug delivery since it can encapsulate a large amount of substances in an internal cavity to isolate the substance from the living bodies. The ferritin particles can be used as a targeting agent since they are readily accumulated to specific organs or tissues (e.g., lymph node, liver, spleen, cancer tissue) and they are readily permeable to blood-brain barrier (BBB). Thus, the complex or salt thereof of the present invention is useful as a product such as a pharmaceutical or reagent, since it can distribute a function as a drug, a means for drug delivery, and/or a targeting agent to one or both of the antibody and human ferritin particles.

### Brief Description of Drawings

FIG. 1 is a diagram illustrating (A) an amino acid sequence of a natural human ferritin H chain (with methionine at position 1) (SEQ ID NO: 1), (B) a nucleotide sequence of a polynucleotide encoding a natural human ferritin H chain (with methionine at position 1) (SEQ ID NO: 2), and (C) an amino acid sequence of a natural human ferritin H chain (without methionine at position 1) (SEQ ID NO: 3).
FIG. 2 is a diagram illustrating ESI-TOFMS analysis of a natural human ferritin H chain specifically modified with ethyl maleimide (six equivalents).
FIG. 3 is a diagram illustrating analysis (MS spectrum) of a modified site of a natural human ferritin H chain specifically modified with ethyl maleimide.
FIG. 4 is a diagram illustrating analysis (CID spectrum) of a modified site of a natural human ferritin H chain specifically modified with ethyl maleimide.
FIG. 5 is a diagram illustrating ESI-TOFMS analysis of a natural human ferritin H chain specifically modified with p-azidophenacyl bromide (six equivalents).
FIG. 6 is a diagram illustrating ESI-TOFMS analysis of a natural human ferritin H chain specifically modified with p-azidophenacyl bromide (12 equivalents).
FIG. 7 is a diagram illustrating analysis (MS spectrum) of a modified site of a natural human ferritin H chain specifically modified with p-azidophenacyl bromide.
FIG. 8 is a diagram illustrating analysis (CID spectrum) of a modified site of a natural human ferritin H chain specifically modified with p-azidophenacyl bromide.
FIG. 9 is a diagram illustrating a comparison among peak area values at positions 91, 103, and 131 of a human ferritin H chain based on an analysis result of a modified site of a human ferritin H chain by LC-MS/MS.
FIG. 10 is a diagram illustrating ESI-TOFMS analysis of a natural human ferritin H chain specifically modified with p-azidophenacyl bromide (six equivalents).
FIG. 11 is a diagram illustrating ESI-TOFMS analysis of a natural human ferritin H chain specifically modified with DBCO-PEG4-maleimide (six equivalents).
FIG. 12 is a diagram illustrating ESI-TOFMS analysis of natural human ferritin H chains specifically modified with 1,3-dichloroacetone (six equivalents) and aminooxy-PEG5-azide (ten equivalents).
FIG. 13 is a diagram illustrating ESI-TOFMS analysis of a DBCO-modified antibody.
FIG. 14 is a diagram illustrating ESI-TOFMS analysis of a DBCO-PEG-modified antibody.
FIG. 15 is a diagram illustrating a migration image of an antibody-human ferritin complex by SDS-PAGE.
FIG. 16 is a diagram illustrating a transmission electron microscope (TEM) image of the antibody-human ferritin complex.
FIG. 17 is a diagram illustrating a migration image of the antibody-human ferritin complex by SDS-PAGE.

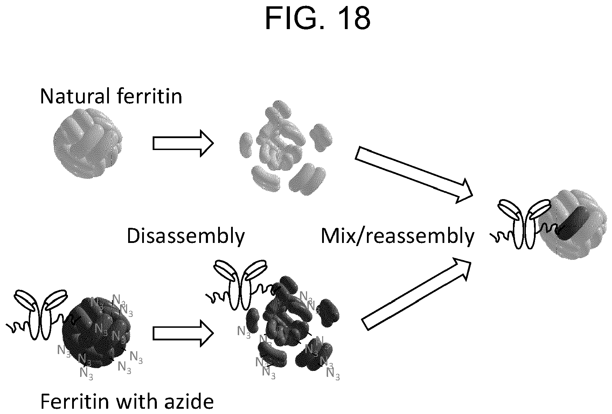

FIG. 18 is a diagram illustrating dissociation process of the antibody-human ferritin complex.
FIG. 19 is a diagram illustrating ESI-TOFMS analysis of a DBCO-modified antibody-human ferritin complex.
FIG. 20 is a diagram illustrating ESI-TOFMS analysis of a DBCO-PEG-modified antibody-human ferritin complex.
FIG. 21 is a diagram illustrating a reverse phase chromatography analysis of a mosaic ferritin.
FIG. 22 is a diagram illustrating reverse phase chromatography analysis of a DBCO-modified antibody-mosaic ferritin complex.
FIG. 23 is a diagram illustrating a change in the amount of monomers in the mosaic ferritin by antibody modification reactions.
FIG. 24 is a diagram illustrating a migration image of an antibody-human ferritin complex by SDS-PAGE.

### Embodiments for carrying out the invention

The present invention provides a complex or salt thereof comprising (A) one IgG antibody and (B) two human ferritin particles comprising one or more human ferritin H chains linked thereto. The two human ferritin particles each comprise one or more human ferritin H chains.

### (I) Human ferritin particle

### (I-1) Human ferritin particle comprising human ferritin H chain

The human ferritin particle can form a 24-mer comprising 24 ferritin monomers (H-chain and L-chain). Naturally occurring ferritin particles in humans are a hetero 24-mer which is usually a mixture of H and L chains, and the experimentally prepared ferritin particles are usually a homo 24-mer. This is due to the burden of separately preparing and mixing the H-chain and L-chain ferritin particles when the hetero 24-mer is prepared in the experiment, as a homo 24-mer is prepared from the standpoint of reducing the experimental burden. Thus, the human ferritin particle comprising one or more human ferritin H chains may be a homo 24-mer comprising only human ferritin H chains, or a hetero 24-mer comprising human ferritin H chains and L chains. In this case, the human ferritin L chain may comprise mutations as described below for the human ferritin H chain (e.g., mutations that further include a functional peptide, and modifications of the N-terminus and C-terminus). In a simple preparation aspect, the human ferritin particle is preferablyh a homo 24-mer comprising 24 human ferritin H chains.

The position of the amino acid residue in the human ferritin H chain (e.g., methionine residue at position 1, cysteine residue at position 91, and cysteine residue at position 103) is determined according to the reference position of the natural-type human ferritin H chain. As used herein, the term "natural-type" indicates that the amino acid sequence is natural-type. Thus, a "natural-type human ferritin H chain" refers to a human ferritin H chain having a natural-type amino acid sequence. On the other hand, the "modified human ferritin H chain" described below means a human ferritin H chain with modification of the side chain of the amino acid residue constituting the human ferritin H chain and differs from the "natural-type human ferritin H chain" which does not mean the modification of the side chain of the amino acid residue. The natural-type human ferritin H chain is typically a polypeptide comprising the amino acid sequence of SEQ ID NO: 1. In the present invention, the natural-type human ferritin H chain includes not only a polypeptide comprising the amino acid sequence of SEQ ID NO: 1, but also a naturally occurring variant thereof. Preferably, the natural-type human ferritin H chain is a polypeptide comprising the amino acid sequence of SEQ ID NO: 1.

The human ferritin H chain contained in the modified ferritin of the present invention may be a natural-type human ferritin H chain which may lack the methionine residue at position 1. A natural-type human ferritin H chain which does not lack the methionine residue at position 1 corresponds to a polypeptide comprising the amino acid sequence of SEQ ID NO: 1. A natural-type human ferritin H chain which lacks the methionine residue at position 1 corresponds to a polypeptide comprising the amino acid sequence of SEQ ID NO: 3.

In a specific embodiment, the human ferritin H chain may be the following:
(A) A protein comprising the amino acid sequence of SEQ ID NO: 1 or SEQ ID NO: 3;
(B) A protein comprising an amino acid sequence comprising a mutation of one or several amino acid residues, which is selected from the group consisting of substitution, deletion, insertion, and addition of amino acid residues in the amino acid sequence of SEQ ID NO: 1 or SEQ ID NO: 3;
(C) A protein comprising an amino acid sequence having 90% or more identity to the amino acid sequence of SEQ ID NO: 1 or SEQ ID NO: 3 and having an ability to form a 24-mer.

In protein (B), one or several amino acid residues can be modified by one, two, three, or four kinds of mutations selected from the group consisting of deletion, substitution, addition and insertion of amino acid residues. The mutations of amino acid residues may be introduced into one region in the amino acid sequence, but may be introduced into a plurality of different regions. In the present invention, substitution of amino acid residues from amino acid residues other than cysteine residues is not intended. Also, for the addition and insertion of amino acid residues. addition and insertion of a cysteine residue or a cysteine residue-containing region are not intended. The term "one or several" refers to the number that does not impair the ability to form a 24-mer. The number indicated by the term "one or several" is, for example, 1 to 20, preferably 1 to 15, more preferably 1 to 10, even more preferably 1 to 5 (e.g., 1, 2, 3, 4, or 5).

In the protein (C), the degree of identity to an amino acid sequence of interest is preferably 92% or more, more preferably 95% or more, still more preferably 97% or more, and most preferably 98% or more or 99% or more. The % identity of the amino acid sequence can be calculated using GENETYX Ver 13.1.1 software manufactured by GENETYX Corporation, and using a numerical value obtained by performing muscle alignment, ClustalW alignment, or Multiple sequence alignment using the entire length of a polypeptide portion encoded by ORF, and then performing calculation with setting of Gaps are taken into account.

While the position of the amino acid residue to which the mutation is to be introduced in the amino acid sequence will be apparent to a person ordinary skill in the art, it may be specified with reference to sequence alignment. Specifically, a person ordinary skill in the art can recognize a correlation between structure and function by 1) comparing a plurality of amino acid sequences, 2) revealing regions that are relatively conserved, and regions that are not relatively conserved, and then 3) predicting regions that may play an important role in the function and regions that may not play an important role in the function from the regions that are relatively conserved, and the regions that are not relatively conserved, respectively. Thus, a person ordinary skill in the art can identify the positions to which mutations should be introduced in the amino acid sequence by utilizing sequence alignment, and can also use known secondary and tertiary structure information to identify the positions of the amino acid residue to which the mutations should be introduced in the amino acid sequence. It is also preferred to introduce mutations into amino acid residues in C-terminal regions and other regions located within the cage-like structure of ferritin under normal circumstances in terms of avoiding the risk of developing immunogenicity (amino acid residues which indicates low risk of developing immunogenicity since they are not exposed to the surface). Also, for positions that are not exposed to ferritin surfaces, mutations (e.g., substitutions, insertions, and additions to N-terminal regions) of the cysteine residue or cysteine residue-containing peptide may be introduced, but it is also preferred that mutations (e.g., substitutions, insertions, and additions to N-terminal regions) of the cysteine residue or cysteine residue-containing peptide are not introduced.

When the amino acid residue is mutated by substitution, the substitution of the amino acid residue may be a conservative substitution. As used herein, the term "conservative substitution" refers to replacing a given amino acid residue with an amino acid residue having a similar side chain. A family of amino acid residues having similar side chains is well known in the art. For example, such families include amino acids having basic side chains (e.g., lysine, arginine, histidine), amino acids having acidic side chains (e.g., aspartic acid, glutamic acid), amino acids having non-charged polar side chains (e.g., glycine, asparagine, glutamine, serine, threonine, tyrosine, cysteine), amino acids having nonpolar side chains (e.g., alanine, valine, leucine, isoleucine, proline, phenylalanine, methionine, tryptophan), amino acids having β-branched side chains (e.g., threonine, valine, isoleucine), amino acids having aromatic side chains (e.g., tyrosine, phenylalanine, tryptophan, histidine), amino acids having a hydroxyl group (e.g., alcholic and phenolic) (e.g., serine, threonine, tyrosine), and amino acids having sulfur-containing side chains (e.g., cysteine, methionine). Preferably, the conservative substitution of the amino acid may be a substitution between aspartic acid and glutamic acid, a substitution among arginine, lysine and histidine, a substitution between tryptophan and phenylalanine, a substitution between phenylalanine and a valine, a substitution among leucine, isoleucine and alanine, and a substitution between glycine and alanine.

In a specific embodiment, the human ferritin H chain in the proteins of (B) and (C) may be a human ferritin H chain further comprising a functional peptide that does not contain cysteine residues. In the present invention, genetic modifications for further comprising functional peptides are permitted because genetic modifications other than genetic modifications for introduce a modifying group on the ferritin surface are permitted. The N-terminus of the ferritin monomer of higher organisms is exposed on the surface of the 24-mer. In the human ferritin H chain which is a ferritin monomer for higher organisms, a variety of functional peptides can be inserted at its N-terminus, while maintaining the 24-mer formation. Also, the ferritin monomers of higher organisms have six α-helices that are highly conserved among various higher organisms. In the human ferritin H chain which is a ferritin monomer for higher organisms, as six α-helices, 1) a first region consisting of amino acid residues at positions 15-42 (also referred to as the region A), 2) a second region consisting of amino acid residues 50-77 (also referred to as the region B), 3) a third region consisting of amino acid residues at positions 97-124 (also referred to as the region C), 4) a fourth region consisting of amino acid residues at positions 128-137 (also referred to as the region D), 5) a fifth region consisting of amino acid residues at position 139-159 (also referred to as the region D), and 6) a sixth region consisting of amino acid residues at position 165-174 (also referred to as the region E). The region between these α-helices is referred to as a flexible linker region, and it has been reported that a variety of functional peptides can be inserted into the flexible linker regions while maintaining the ability to form 24-mer (e.g., WO2019/163871; US Patent Application Publication No. 2016/0060307; Jae Og Jeon et al., ACS Nano (2013), 7(9), 7462-7471; Sooji Kim et al., Biomaterials (2016), 17(3), 1150-1159; Young Ji Kang et al, Biomaterials (2012), 13(12), 4057-4064). For example, the α-helix of the region B and the region C in the ferritin monomer is well known in the art, and a person ordinary skill in the art can appropriately determine the position of the α-helix in region B and the region C in ferritin monomers from various organisms. Thus, the flexible linker region between the α-helices of the regions B and C into which the functional peptide is inserted in the present invention is also well known in the art and can be identified as appropriate by a person ordinarly skilled in the art. For example, as such an insertion position of a functional peptide to a human ferritin H chain, positions 78-96 (preferably positions 83-91) which are flexible linker regions between the second and third regions, can be suitably utilized (e.g., WO2019/163871).

For the functional peptide, it is possible to use a peptide that allows an arbitrary function to be added to the aimed protein when fused to the aimed protein. Examples of such a functional peptide include a peptide capable of binding to a target material, a protease-degrading peptide, a cell-permeable peptide, and a stabilizing peptide.

The functional peptide inserted into the above-described region may be only one peptide having the desired function or may be a plurality (e.g., several such as two, three, or four) peptides of the same or different type having the desired function. Where the functional peptide is a plurality of peptides as described above, the plurality of functional peptides may be inserted in any order and fused with the ferritin monomer. The fusion can be achieved via amide bonds. The fusion may be achieved directly by amide bonding, or indirectly by amide bonds via peptides (peptide linkers) consisting of one amino acid residue or several (e.g., 2 to 20, preferably 2 to 10, more preferably 2, 3, 4 or 5) amino acid residues. Since a variety of peptide linkers are known, such peptide linkers can also be used in the present invention. Preferably, the total length of the peptide inserted in the above-described region is less than or equal to 20 amino acid residues.

As the functional peptide, when a peptide capable of binding to a target material is used, examples of the target material includes a biological organic molecule, a material capable of interacting with a tag for protein purification (e.g., histidine tag, maltose binding protein tag, glutathione-S-transferase) (e.g., nickel, maltose, glutathione), and a labeling substance (e.g., radioactive material, fluorescent material, dye). The ferritin is known to be incorporated into transferrin receptor-presenting cells (e.g., L. Li et al. Proc Natl Acad Sci USA. 2010; 107(8): 3505-10). When the peptide capable of binding to a biological organic molecule is used as the functional peptide, the ferritin can be delivered to cells and organs that express a biological organic molecule where the ferritin does not originally bind to the cells and organs.

Examples of the biological organic molecules include proteins (e.g., oligopeptide or polypeptide), nucleic acids (e.g., DNA, RNA, nucleosides, nucleotides, oligonucleotides or polynucleotides), saccharides (e.g., monosaccharides, oligosaccharides or polysaccharides) and lipids. The biological organic molecule may also be a cell surface antigen (e.g., a cancer antigen, a heart disease marker, a diabetes marker, a neurological disease marker, an immune disease marker, an inflammatory marker, a hormone, or an infectious disease marker). The biological organic molecule may also be a disease antigen (e.g., a cancer antigen, a heart disease marker, a diabetes marker, a neurological disease marker, an immune disease marker, an inflammatory marker, a hormone, or an infectious disease marker). As the peptide capable of binding to such a biological organic molecule, various peptides have been reported. For example, various peptides such as peptides capable of binding to protein (see, e.g., F. Danhier et al., Mol. Pharmaceutics, 2012, vol. 9, No. 11, p. 2961; C-H. Wu et al., Sci. Transl. Med., 2015, Vol. 7, No. 290, 290-91; L. Vannucci et al., Int. J. Nanomedicine 2012, Vol. 7, p. 1489; J. Cutrera et al., Mol. Ther. 2011, Vol. 19 (8), p. 1468; R. Liu et al., Adv. Drug Deliv. Rev. 2017, Vol. 110-111, p. 13), peptides capable of binding to nucleic acid (see e.g., R. Tan et al., Proc. Natl. Acad. Sci. USA, 1995, vol. 92, p. 5282; R. Tan et al., Cell, 1993, vol. 73, p 1031; R. Talanian et al., Biochemistry 1992, Vol. 31, p. 6871), peptides capable of binding to saccharide (see, e.g., K. Oldenburg et al., Proc. Natl. Acad. Sci. USA, 1992, vol. 89, No. 12, p. 5393-5397; K. Yamamoto et al., J. Biochem., 1992, vol. 111, p. 436; A. Baimiev et al., Mol. Biol. (Moscow), 2005, vol. 39, No. 1, p. 90), and peptides capable of binding to lipid (see, e.g., O. Kruse et al., B Z. Naturforsch., 1995, Vol. 50c, p. 380; O. Silva et al., Sci. Rep., 2016, Vol. 6, 27128; A. Filoteo et al., J. Biol. Chem., 1992, vol. 267, No. 17, p. 11800) have been reported.

When the protease-degrading peptide is used as the functional peptide, examples of the protease include cysteine protease such as caspase and cathepsin (D. McIlwain1 et al., Cold Spring Harb Perspect Biol., 2013, vol. 5, a008656; V. Stoka et al., IUBMB Life. 2005, vol. 57, No. 4-5 p. 347), collagenase (G. Lee et al., Eur J Pharm Biopharm., 2007, vol. 67, No. 3, p. 646), thrombin and Xa factor (R. Jenny et al., Protein Expr. Purif., 2003, vol. 31, p. 1; H. Xu et al., J. Virol., 2010, vol. 84, No. 2, p. 1076), and a virus-derived protease (C. Byrd et al., Drug Dev. Res., 2006, vol. 67, p. 501). Various protease-degrading peptides are known (see, e.g., E. Lee et al., Adv. Funct. Mater., 2015, vol. 25, p. 1279; G. Lee et al., Eur J Pharm Biopharm., 2007, vol. 67, no. 3, p. 646; Y. Kang et al., Biomacromolecules, 2012, vol. 13, No. 12, p. 4057; R. Talanian et al., J. Biol. Chem., 1997, vol. 272, p. 9677; Jenny et al., Protein Expr. Purif., 2003, vol. 31, p. 1; H. Xu et al., J. Virol., 2010, vol. 84, No. 2, p. 1076). In the present invention, various protease-degrading peptides can be used (see, e.g., E. Lee et al., Adv. Funct. Mater., 2015, vol. 25, p. 1279; G. Lee et al., Eur J Pharm Biopharm., 2007, vol. 67, no. 3, p. 646; Y. Kang et al., Biomacromolecules, 2012, vol. 13, No. 12, p. 4057; R. Talanian et al., J. Biol. Chem., 1997, vol. 272, p. 9677; Jenny et al., Protein Expr. Purif., 2003, vol. 31, p. 1; H. Xu et al., J. Virol., 2010, vol. 84, No. 2, p. 1076).

When the stabilizing peptide is used as the functional peptide, various stabilizing peptides can be used (see, e.g., X. Meng et al., Nanoscale, 2011, Vol. 3, No. 3, P. 977; E. Falvo et al., Biomacromolecules, 2016, vol. 17, No. 2, p. 514).

When the cell-permeable peptide is used as the functional peptide, various cell-permeable peptides can be used (see, e.g., Z. Guo et al. Biomed. Rep., 2016, vol. 4, No. 5, p. 528).

For the functional peptide, the peptide capable of binding to the target material is preferred. The peptide capable of binding to the target material is preferably peptide capable of binding to the biological organic molecule, and more preferably peptide capable of binding to protein.

The human ferritin H chain may be modified at N-terminus region and/or C-terminus region thereof. The N-terminus of the ferritin monomer of animals such as the human ferritin monomer can be exposed on a surface of the multimer, while the C-terminus cannot be exposed on the surface. As such, the peptide part added to the N-terminus of the animal ferritin monomer cannot be exposed on the surface of the multimer for interacting with the target material existing outside the multimer, while the peptide part added to the C-terminus of the animal ferritin monomer is not exposed on the surface of the multimer and thus cannot interact with the target material existing outside the multimer (e.g., WO2006/126595). However, it has been reported that the C-terminus of the animal ferritin monomer can be utilized in encapsulation of agents into an internal cavity of the multimer by modification of amino acid residues thereof (see, e.g., Y. J. Kang, Biomacromolecules. 2012, vol. 13(12), 4057).

In a specific embodiment, the human ferritin H chain may have a peptide part that is added to the N-terminus as the modification in the N-terminus region. An example of the peptide part to be added is the functional peptide described above. Other examples of the peptide part to be added include peptide components for improving solubility of the target protein (e.g., Nus-tag), peptide components acting as a chaperone (e.g., trigger factor), peptide components with other functions (e.g., a full-length protein or a part thereof) and linkers. As the peptide part to be added to the N-terminus of the human ferritin H chain, a peptide that is the same as or different from the functional peptide to be inserted at the region between second and third α-helices can be used. From the viewpoint of achievement of the interaction with different target materials, different peptide is preferably used. Preferably, the peptide part to be added to the N-terminus of the human ferritin H chain is the functional peptide described above. The peptide part to be added to the N-terminus is preferably designed such that an amino acid residue (e.g., methionine residue) corresponding to a start codon is contained in the N-terminus. Such a design facilitates translation of the human ferritin H chain.

In another specific embodiment, the modification at the C-terminus region of the human ferritin H chain may be carried out by substituting the amino acid residue at the C-terminus region with a reactive amino acid residue, inserting the reactive amino acid residue at the C-terminus region, or adding the reactive amino acid residue or a peptide containing the same (e.g., a peptide comprising 2 to 12, for example, or preferably 2 to 5 amino acid residues) to the C-terminus. Examples of such a C-terminus region include a region including amino acid residues of the human ferritin H chain at positions 175-183 (preferably positions 179-183). Such a modification allows the reactive amino acid residue to react with a certain substance (e.g., agent and target substance) and thus enables encapsulation of the certain substance into the internal cavity of the multimer through covalent bonds. Examples of such a reactive amino acid residue include cysteine residue having a thiol group, lysine residue having an amino group, arginine residue, asparagine residue, and glutamine residue, and cysteine residue is preferable. Preferably, the modification of the C-terminus region of the fusion protein of the present invention is the addition of the reactive amino acid residue or a peptide containing the same to the C-terminus.

In a preferred embodiment, the human ferritin H chain further comprises the functional peptide, the functional peptide may be a human-derived peptide. When the functional peptide is the human-derived peptide, the risk of developing immunogenicity to humans can be reduced.

### (I-2) Preparation of human ferritin particle comprising one or more human ferritin H chain

The human ferritin particle comprising one or more human ferritin H chain can be obtained by utilizing a host cell that contains polynucleotides encoding the human ferritin H chain and allows production of the human ferritin H chain to form a 24-mer. Examples of the host cell used for producing the human ferritin H chain include cells derived from animals, insects, plants or microorganisms. The animals are preferably mammals or avian species (e.g., chicken), and more preferably mammals. Examples of the mammals include primates (e.g., humans, monkeys, and chimpanzees), rodents (e.g., mice, rats, hamsters, guinea pigs, and rabbits) and livestock and working mammals (e.g., cattle, pigs, sheep, goats, and horses).

In a preferred embodiment, the host cell is a human cell or a cell used for production of a human protein (e.g., Chinese hamster ovary (CHO) cells, and human fetal kidney-derived HEK 293 cells). Such a host cell is preferably used from the viewpoint of clinical application to humans.

In another preferred embodiment, the host cell is a microorganism. From the viewpoint of mass production of the fusion protein and the like, such host cells may be used. Examples of the microorganism include bacteria and fungi. As the bacteria, any bacteria used as the host cells can be used. Examples of the bacterium include bacteria belonging to Bacillus (e.g., Bacillus subtilis), Corynebacterium (e.g., Corynebacterium glutamicum), Escherichia (e.g., Escherichia coli) and Pantoea (e.g., Pantoea ananatis). As the fungi, any fungi used as the host cells can be used. Examples of the fungus include fungi belonging to Saccharomyces (e.g., Saccharomyces cerevisiae) and Schizosaccharomyces (e.g., Schizosaccharomyces pombe). Alternatively, filamentous fungi may be used as the microorganism. Examples of the filamentous fungi include fungi belonging to Acremonium/Talaromyces, Trichoderma, Aspergillus, Neurospora, Fusarium, Chrysosporium, Humicola, Emericella and Hypocrea.

The host cell can be designed so as to include an expression unit containing a promoter operably linked to the polynucleotide in addition to the polynucleotide encoding the human ferritin H chain. The term "expression unit" refers to a unit that contains a certain polynucleotide to be expressed as a protein and the promoter operably linked to the polynucleotide and enables transcription of the polynucleotide and hence production of the protein encoded by the polynucleotide. The expression unit may further include an element such as a terminator, a ribosomal binding site, and a drug resistant gene. The expression unit may be DNA or RNA, and preferably DNA. The expression unit can be included in a genome region (e.g., a natural genome region that is a natural locus, in which a polynucleotide encoding the protein is inherently present, or an unnatural genome region that is not the natural locus), or a non-genomic region (e.g., a cytoplasm) in the microorganism (host cell). The expression units may be included at one or more (e.g., 1, 2, 3, 4 or 5) different positions in the genome region. Examples of specific forms of the expression unit included in the non-genomic region are plasmids, viral vectors, phages or artificial chromosomes.

### (I-3) Modification of human ferritin H chain

The human ferritin H chain contained in human ferritin particles may be a modified human ferritin H chain. The modified human ferritin H chain means a human ferritin H chain in which the side chain of the amino acid residue constituting the human ferritin H chain is modified. The modified human ferritin H chain include a modified human ferritin H chain having a modifying group utilized for linking an IgG antibody, and a modified human ferritin H chain having a modifying group that are not utilized for linking an IgG antibody. The modification of the human ferritin H chain can be carried out in the side chain of any amino acid residue. Preferably, cysteine residues in the side chains of cysteine residues present at position 91 and/or position 103 according to the reference position of the natural-type human ferritin H chain are utilized to highly control the number of human ferritin particles to be linked to the antibody and the regioselectivity of the modification.

In a preferred embodiment, the amino acid residues in the human ferritin H chain modified in the present invention may be cysteine residues present at positions 91 and/or 103 according to the reference position of the natural-type human ferritin H chain. The use of thiol groups in the side chains of such cysteine residues can highly control the number of human ferritin particles to be linked to the antibody as well as liking site of the human ferritin H chain to the antibody and regioselectivity of the modification site by the modifying group. In connection with the linking site of the human ferritin H chain to the antibody and the modification site by the modifying group, the "regioselectively" or "regioselectivity" refers to the localization of the linking site and the modification site in a specific region in the human ferritin H chain (e.g., cysteine residues at position 91 and/or position 103). Such regioselectivity is 50% or more, or may be preferably 60% or more, more preferably 70% or more, even more preferably 80% or more, particularly preferably 90% or more, 95% or more, 96% or more, 97% or more, 98% or more, 99% or more, 99.5% or more, or 1000.

In the human ferritin particle, the cysteine residues at positions 91 and 103 are close to each other. The human ferritin particle can contain one or two modifying groups per human ferritin H chain at such positions. Thus, the human ferritin particle may comprise a modified human ferritin H chain comprising a modifying group of either (A) or (B):
(A) a human ferritin H chain comprising one or two modifying groups specifically and covalently bonded to one or two sulfur atoms in the side chain of one or both cysteine residues at position 91 or position 103; or
(B) a human ferritin H chain comprising one modifying group bridging two sulfur atoms in the side chain of cysteine residues at positions 91 and 103 by specific covalent bonds.

The modified ferritin comprising such a human ferritin H chain can be prepared by setting the molar ratio of human ferritin to the thiol modifying reagent or functional substance described below (human ferritin : thiol modifying reagent or functional substance) to be in the range of, for example, 1:1 to 1:30, preferably 1:1 to 1:20, more preferably 1:1 to 1:15 and reacting them.

In one embodiment, the human ferritin particle can congain 24 or 48 modifying groups. This is because, for the human ferritin particle, a 24-mer can be used that can contain one or two modifying groups per human ferritin H chain in cysteine residues at positions 91 and 103.

The human ferritin particle may further comprise an unmodified human ferritin H chain that do not contain a modifying group. The unmodified human ferritin H chain means a human ferritin H chain in which the side chain of the amino acid residue constituting the human ferritin H chain has not been modified. The human ferritin particle further comprising the unmodified human ferritin H chain can be prepared by reacting a reactive substance (e.g., a thiol modifying reagent and a functional substance) at a low ratio to the ferritin. The human ferritin particles further comprising the unmodified human ferritin H chain can also be prepared by separately preparing human ferritin particles comprising a modified human ferritin H chain comprising a modifying group that specifically and covalently binds to cysteine residues at position 91 and/or position 103, and human ferritin particles comprising the unmodified human ferritin H chains that do not contain the modifying group, and then dissociating and reassociating these particles in a solution comprising these particles at a certain amount ratio. The ferritin particles may be relatively easily dissociated and associated, such that they are in the form of a 24-mer or monomer, or in the co-presence form thereof depending on the conditions such as the solvent comprising them. Thus, by suitably adjusting the conditions such as the solvent comprising the ferritin particles, the 24-mer can be dissociated into monomers, and the dissociated monomers can be reassociated into the 24-mer. Such dissociation and reassociation have been utilized in encapsulating various substances (e.g., drugs described below) in an internal cavity of the ferritin particles (see, e.g., WO 2020/090708; Chinese Patent Application Publication 106110333; Journal of Controlled Release 196(2014) 184-196; Biomacromics 2016, 17, 514-522; Proc Natl Acad Sci USA. 2014 111(41): 14900-5). When the human ferritin particle further comprises the unmodified human ferritin H chain, the molar ratio of the modified human ferritin H chain containing the modifying group and the unmodified human ferritin H chain not containing the modifying group in the human ferritin particle can be controlled depending on the ratio of the amount of the reactive substance to the ferritin particle, or the ratio of these before reassociation in the solution.

In one embodiment, the modifying group may comprise a reactive group. In this case, the human ferritin particle comprises a modified human ferritin H chain comprising a modifying group comprising a reactive group, which is linked to a cysteine residue at position 91 and/or position 103. The human ferritin particle comprising such a modified human ferritin H chain can be prepared by reacting a modified human ferritin H chain or a human ferritin particle comprising it (preferably, a human ferritin particle comprising such a modified human ferritin H chain) with a thiol modifying reagent as described below.

The reactive group may be suitably set to react with the functional substance. Thus, the reactive group may comprise a moiety capable of reacting with a given functional group in the functional substance (the functional substance may be derivatized to have a certain functional group capable of reacting with the reactive group). The number of reactive groups contained in the modifying group is single or plural, for example, 1 to 5, preferably 1 to 3, more preferably 1 or 2, even more preferably 1. When the number of reactive groups contained in the modifying group is plural, the plural reactive groups may be the same or different. For example, when reactions with a plurality of the same functional substances are desired, the same reactive groups are preferred in terms of adoption of simple structures and the like. On the other hand, when a reaction with a plurality of different functional substances is desired, different reactive groups are preferred in terms of ensuring the selectivity of the reaction and the like.

In a certain embodiment, the reactive group may be a bioorthogonal functional group to a protein. In such a case, the reaction between the ferritin H chain and the functional substance can be promoted while suppressing the reaction between ferritin monomers. The bioorthogonal functional group refers to a group having such reaction selectivity that the bioorthogonal functional group cannot react with a biological component (for example, an amino acid, a nucleic acid, a lipid, a sugar, or a phosphoric acid) or hardly reacts with the biological component but easily reacts with a component other than the biological component. The bioorthogonal functional group is well known in the technical field concerned (see, for example, Sharpless K. B. et al., Angew. Chem. Int. Ed. 40, 2004 (2015); Bertozzi C. R. et al., Science 291, 2357 (2001); and Bertozzi C. R. et al., Nature Chemical Biology 1, 13 (2005)). Therefore, the bioorthogonal functional group for a protein means a group having such reaction selectivity that the bioorthogonal functional group cannot react with a side chain of 20 types of natural amino acid residues constituting the protein or hardly reacts with the side chain but easily reacts with a target other than the side chain. The naturally occurring 20 amino acids constituting the protein are alanine (A), asparagine (N), cysteine (C), glutamine (Q), glycine (G), isoleucine (I), leucine (L), methionine (M), phenylalanine (F), proline (P), serine (S), threonine (T), Tryptophan (W), tyrosine (Y), valine (V), aspartic acid (D), glutamic acid (E), arginine (R), histidine (H), and lysine (L). Among these naturally occurring 20 amino acids, glycine which contain no side chain (i.e., hydrogen atom) and alanine, isoleucine, leucine, phenylalanine and valine whose side chain is a hydrocarbon group (i.e., which does not contain a heteroatom selected from the group consisting of a sulfur atom, a nitrogen atom, and an oxygen atom) are inert to normal reactions. Thus, the bioorthogonal functional group to the protein is a functional group that cannot react with side chains of those amino acids having the side chains that are inert to normal reactions, but also to side chains of asparagine, glutamine, methionine, proline, serine, threonine, tryptophan, tyrosine, aspartic acid, glutamic acid, arginine, histidine and lysine, as well as side chains of cysteine (e.g., when the cysteine residues at position 91 and/or 103 of human ferritin H chain are not sufficiently modified).

Examples of the bioorthogonal functional group to the protein include one or more partial structures selected from the group consisting of a maleimide moiety, an azide moiety, a ketone moiety, an aldehyde moiety, a thiol moiety, an alkene moiety (in other words, it may have a vinyl (ethenyl) moiety or vinylene (ethenylene) moiety which is the smallest unit having double bond between carbon atoms. the same applies hereinafter), an alkyne moiety (in other words, it may have an ethynyl moiety or ethynylene moiety which is the smallest unit having triple bond between carbon atoms. the same applies hereinafter), a halogen moiety (e.g., fluorinated moiety, chloride moiety, bromide moiety, iodide moiety), a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety (e.g., a carbonyl moiety having a fluorine atom, chlorine atom, bromine atom or iodine atom at position α), an isonitrile moiety, a sydnone moiety, and a selenium moiety. The proteins include proteins that may contain free thiols in the side chains of cysteine residues (e.g., proteins other than antibodies) and proteins that may not contain free thiols (e.g., antibodies, proteins that do not contain cysteine residues, and ferritin comprising human ferritin H chains in which cysteine residues at position 91 and/or 103 are sufficiently modified as provided in the present invention). In the proteins that do not contain free thiol, the thiol functions as a bioorthogonal functional group. Thus, in the present invention, the bioorthogonal functional group to the protein includes a thiol moiety in principle. However, when the cysteine residues at position 91 and/or 103 are not sufficiently modified (e.g., when the average number of modifications of cysteine residues per human ferritin H chain is one), the bioorthogonal functional group to the protein does not include the thiol moiety.

The reactive group can also be set to specifically react with a given functional group in the functional substance depending on the reactive group and the type of functional substance that can be subsequently reacted (e.g., peptide, protein, nucleic acid, lipid, small organic compound).

The reaction for producing a modified human ferritin H chain comprising a reactive group as described above can be performed as appropriate under conditions that cannot cause denaturation or degradation (e.g., cleavage of amide bonds) (mild conditions). For example, such a reaction can be performed in a suitable reaction system such as a buffer under temperature conditions that may not cause denaturation or degradation of the ferritin (e.g., about 15-60°C, preferably 15-45°C). The pH of the buffer is, for example, 3.0-12.0, preferably 5.0-9.0, more preferably 6.0-8.0. The buffer may comprise a component such as a catalyst. The reaction time is, for example, from 1 minute to 20 hours, preferably from 10 minutes to 15 hours, more preferably from 20 minutes to 10 hours, even more preferably from 30 minutes to 8 hours.

In another embodiment, the modifying group may comprise a functional substance. In this case, the human ferritin particle comprises a modified human ferritin H chain comprising a modifying group comprising a functional substance, which is linked to a cysteine residue(s) at position 91 and/or position 103.

The functional substance is not limited to a particular substance as long as it is a substance imparting any function to the ferritin; examples thereof include drugs, labelling substances, and stabilizers; preferred are drugs and labelling substances. The functional substance may be a single functional substance or a substance in which two or more functional substances are linked with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., 35^{S}), radioisotopes of yttrium (e.g., ⁹⁰Y), radioisotopes of technetium (e.g., ^{99m}Tc), radioisotopes of indium (e.g., ¹¹¹In), radioisotopes of an iodide atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubricin.

The targeting substance is an affinity substance for a target (e.g., tissue, cell, substance). Examples of the targeting substance include antibodies to a target substance or fragments thereof capable of binding to a target substance, ligands capable of binding to receptors, proteins capable of forming complexes (e.g., adapter proteins), proteins capable of binding to sugar chains (lectins), sugar chains capable of binding to proteins, and nucleic acids capable of binding to complementary sequences (e.g., natural nucleic acids such as DNA and RNA, or artificial nucleic acids).

The labelling substance is a substance that enables detection of a target (e.g., a tissue, a cell, or a substance). Examples of the labelling substance include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl) Duthenium, and luminol), and radioisotopes (e.g., those described above) or substances comprising them.

The stabilizer is a substance that enables stabilization of the ferritin. Examples of the stabilizer include diols, glycerin, nonionic surfactants, anionic surfactants, natural surfactants, saccharides, and polyols.

The functional substance may also be a peptide, a protein (e.g., an antibody), a nucleic acid (e.g., DNA, RNA and artificial nucleic acid), a small organic compound (e.g., small organic compound described below), a chelator, a sugar chain, a lipid, a high polymer, or a metal (e.g., gold).

The human ferritin particle comprising a modified human ferritin H chain comprising a modifying group comprising a functional substance can be prepared by reacting the human ferritin particle with the functional substance. The functional substance used in such a reaction is a reactive substance having at least one reaction site to a thiol group and having an ability to add a modifying group comprising a functional substance to a human ferritin by a reaction. For the functional substance used in such a reaction, any functional substance having a functional group that is readily reactive with a thiol group can be used. Alternaturally, if the functional substance does not have a functional group that is readily reactive with a thiol group, a functional substance derivatized to have such a functional group can be used. In view of increased reaction efficiency, the functional substance (which may be derivatized) preferably comprises one or more partial structures selected from the group consisting of a maleimide moiety, a benzyl halide moiety, α haloamide moiety, α haloketone moiety an alkene moiety, an alkyne moiety, a fluoroaryl moiety, a nitroaryl moiety, a methylsulfonyloxadiazole moiety, and a disulfide moiety.

The human ferritin particle comprising a modified human ferritin H chain comprising a modifying group comprising a functional substance can also be prepared by reacting the human ferritin with a thiol modifying reagent and then further reacting a human ferritin particle comprising a modified human ferritin H chain comprising a reactive group obtained by the reaction with a functional substance. The step of reacting the human ferritin particle with the thiol modifying reagent can be performed as described above. The functional substance used in the step of further reacting the human ferritin particles comprising a modified human ferritin H chain comprising a reactive group with the functional substance is a reactive substance having at least one reaction site to a reactive group in a modified human ferritin H chain comprising a reactive group, and having an ability to add a modifying group comprising a functional substance to the human ferritin by a reaction. For the functional substance used in such a reaction, any functional substance having a functional group that is readily reactive with a reactive group can be used. Alternaturally, if the functional substance does not have a functional group that is readily reactive with a reactive group, a functional substance derivatized to have such a functional group may be used. Accordingly, the functional group in the functional substance utilized for the reaction can be determined appropriately depending on the type of reactive group.

The derivatization is common technical knowledge in the art (e.g., WO2004/010957, US2006/0074008, US2005/02386649). For example, the derivatization may be carried out using a cross-linking agent as described above. Alternatively, the derivatization may be carried out using a specific linker having a desired functional group. For example, such a linker may be capable of separating a functional substance and an antibody in a suitable environment (e.g., intracellular or extracellular) by cleavage of the linker. Such linkers include, for example, peptidyl linkers that are degraded by specific proteases [e.g., intracellular proteases (e.g., proteases present in lysosomes, or endosomes), extracellular proteases (e.g., secretory proteases)] (e.g., USP6,214,345; Dubowchik et al., Pharma. Therapeutics 83: 67-123 (1999)), a linker that can be cleaved at a locally acidic site present in the living body (e.g., USP5,622,929, 5,122,368; 5,824,805). The linker may be self-immolative (e.g., WO02/083180, WO04/043493, WO05/1192919). In the present invention, the derivatized functional substance is also simply referred to as "functional substance".

In a specific embodiment, the functional substance may be a functional substance having a functional group that is readily reactive with a reactive group that is a bioorthogonal functional group to the protein, or a functional substance derivatized to have a functional group that is readily reactive with the reactive group. The functional group that is readily reactive with the bioorthogonal functional group can also vary depending on the specific types of bioorthogonal functional group. One skilled in the art can appropriately select a suitable functional group as a functional group that is readily reactive with the bioorthogonal functional group (e.g., Boutureira et al, Chem Rev. 2015, 115, 2174-2195). The functional group that is readily reactive with the bioorthogonal functional group include, but are not limited to, a maleimide group and disulfide group when the bioorthogonal functional group correspond to the thiol moiety; an azide group when the bioorthogonal functional group corresponds to the alkyne moiety; and a hydrazine group when the bioorthogonal functional group corresponds to the aldehyde moiety or ketone moiety.

A reaction for producing the human ferritin particle comprising a modified human ferritin H chain comprising a modifying group comprising a functional substance can be performed appropriately under conditions that cannot cause denaturation or degradation of the ferritin (e.g., cleavage of amide bonds) (mild conditions). For example, such a reaction can be performed in a suitable reaction system such as a buffer under temperature conditions that may not cause denaturation or degradation of the ferritin (e.g., about 15-60°C, preferably 15-45°C). The pH of the buffer is, for example, 3.0-12.0, preferably 5.0-9.0, more preferably 6.0-8.0. The buffer may comprise a component such as a catalyst. The reaction time is, for example, from 1 minute to 20 hours, preferably from 10 minutes to 15 hours, more preferably from 20 minutes to 10 hours, even more preferably from 30 minutes to 8 hours.

The human ferritin particles may have a substance in an internal cavity. Encapsulation of substances into the internal cavity of the human ferritin particle can be accomplished by utilizing the properties of the human ferritin particle capable of incorporating the substances. The human ferritin particle form a cage-like structure having an internal cavity with an outer diameter of about 12 nm (inner diameter of 7 nm). Thus, the size of the substances may be of a size that allows it to be encapsulated into such an internal cavity. It has been reported that various inorganic substances and organic substances can be encapsulated in the internal cavity of human ferritin particles (e.g., WO 2020/090708; Chinese Patent Application Publication 106110333; Journal of Controlled Release 196(2014) 184-196; Biomacromics 2016, 17, 514-522; Proc Natl Acad Sci USA. 2014 111(41): 14900-5). The encapsulation of the substances into the internal cavity of the human ferritin particle can occur before and after the human ferritin particle is subjected to a modification reaction. For the substances, small organic compounds are preferred. The small organic compound refer to an organic compound having a molecular weight of 1500 or less. The small organic compound is a natural or synthetic compound. The small organic compound may have a molecular weight of 1200 or less, 1000 or less, 900 or less, 800 or less, 700 or less, 600 or less, 500 or less, 400 or less, or 300 or less. The molecular weight of the small organic compound may also be 30 or more, 40 or more, or 50 or more. The small organic compound may be a drug or labeling substance as described above. Examples of the small organic compound include amino acids, oligopeptides, vitamins, nucleosides, nucleotides, oligonucleotides, monosaccharides, oligosaccharides, lipids, fatty acids, and salts thereof

The human ferritin particles may be provided as a set of different types of modified ferritin comprising a plurality of different types (e.g., two, three, or four) substances when having the substances in the internal cavity. For example, when the human ferritin particles are provided as a set of two modified ferritin having two substances, such a set may be obtained by combining a first modified ferritin having a first substance and a second modified ferritin having a second substance different from the first substance, each separately prepared.

Confirmation of the production of the human ferritin particles comprising a modified human ferritin H chain can be performed by, for example, electrophoresis, chromatography (e.g., gel filtration chromatography, ion exchange chromatography, reverse phase column chromatography, HPLC), and/or mass spectrometry, depending on the type and molecular weight of the modifying group. Confirmation of the regioselectivity of the modification can be performed, for example, by peptide mapping. The peptide mapping can be performed, for example, by protease (e.g., trypsin, chymotrypsin,) treatment and mass spectrometry. For the protease, an endoprotease is preferred. Examples of such an endoprotease include trypsin, chymotrypsin, Glu-C, Lys-N, Lys-C, and Asp-N. The modified human ferritin H chain or human ferritin particles comprising it can be purified by any method such as chromatography (e.g., chromatography as described above, and affinity chromatography).

### (II) IgG antibody and salt thereof

The types of the antibodies are not particularly limited, and they are derived from animals such as mammals or birds (e.g., chickens), and are preferably derived from mammals. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The types of the IgG antibodies may be a polyclonal antibody or a monoclonal antibody, and the monoclonal antibody is preferred. Examples of the monoclonal antibody include a chimeric antibody, a humanized antibody, or a human antibody. Preferably, the IgG antibody is a chimeric monoclonal antibody comprising a human IgG-derived portion, a humanized monoclonal antibody comprising a human IgG-derived portion, or a human monoclonal antibody. Examples of IgG isotypes include IgG1, IgG2, IgG3 and IgG4.

The IgG antibody may also be an IgG antibody with a certain sugar chain added (e.g., an antibody modified so as to have a sugar chain-binding consensus sequence such as an N-type sugar chain-binding consensus sequence), a bi-specific IgG antibodies, an IgG Fc region protein, or an IgG Fc-fusion protein.

The IgG antibody may further be a full-length IgG antibody, or an IgG antibody fragment comprising variable region and a CH1 domain and CH2 domain (all or partial). Preferably, the IgG antibody is the full-length IgG antibody.

As an antigen of the IgG antibody, any antigen can be used. Examples of such an antigen include a protein [which comprises an oligopeptide and a polypeptide, and may be a protein modified with a biomolecule such as a sugar (e.g., a glycoprotein)], a sugar chain, a nucleic acid, and a small compound. The IgG antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the IgG antibody may be a disease target protein. Examples of the disease target protein include the following.

### (1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

### (2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, and Siglec-15

### (8) Blood Diseases

vWF, Factor IXa, Factor X, IFNγ, C5, BMP-6, Ferroportin, and TFPI

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the IgG antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

The positions of an amino acid residue in the IgG antibody and the position of a constant region of a heavy chain (e.g., CH2 domain) are in accordance with EU numbering (see, http://www.imgt.org/IMGTScientificChart/Numbering/Hu_IGHGnb er. html). For example, when human IgG is a target, a lysine residue at position 246 corresponds to an amino acid residue at position 16 of a human IgG CH2 region, a lysine residue at position 248 corresponds to an amino acid residue at position 18 of a human IgG CH2 region, a lysine residue at position 288 corresponds to an amino acid residue at position 58 of a human IgG CH2 region, a lysine residue at position 290 corresponds to an amino acid residue at position 60 of a human IgG CH2 region, and a lysine residue at position 317 corresponds to an amino acid residue at position 87 of a human IgG CH2 region. The notation at position 246/248 indicates that a lysine residue at position 246 or position 248 is a target. The notation at position 288/290 indicates that a lysine residue at position 288 or position 290 is a target.

In one embodiment, the IgG antibody may be modified regioselectively at a specific amino acid residue. In connection with the IgG antibody, "regioselective" or "regioselectivity" refers to a state in which even though a specific amino acid residue is not present locally in a specific region in the IgG antibody, a certain structural unit capable of binding to the specific amino acid residue in the IgG antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%. For example, a method for regioselectively modifying a specific amino acid residue at a certain position in an antibody is described in WO 2018/199337 A, WO 2019/240288 A, WO 2019/240287 A, and WO 2020/090979 A. As such a specific amino acid residue, an amino acid residue (e.g., a lysine residue, an aspartic acid residue, a glutamic acid residue, an asparagine residue, a glutamine residue, a threonine residue, a serine residue, a tyrosine residue, or a cysteine residue) having a side chain that is easily modified (e.g., an amino group, a carboxy group, an amide group, a hydroxy group, or a thiol group) can be used. However, a lysine residue having a side chain comprising an amino group, a tyrosine residue having a side chain comprising a hydroxy group, a serine residue, a threonine residue, or a cysteine residue having a side chain comprising a thiol group may be preferred, a lysine residue may be more preferred (i.e., among lysine residue at the position 246/248, lysine residue at the position 288/290, and lysine residue at the position 317, two lysine residues may be double-modified regioselectively, and the three lysine residues may be triple-modified regioselectively).

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### (III) Linking human ferritin particle and IgG antibody

In a specific embodiment, the human ferritin particle and the IgG antibody may be linked regioselectively. In this case, the IgG antibody of the present invention can be represented by the following formula (1):

X1-L1-Y-L2-X2 (1)

wherein
X1 and X2 each indicate a human ferritin particle comprising one or more human ferritin H chains,
Y indicates an IgG antibody,
L1 and L2 each indicate a linker, and
the two human ferritin particles indicated by X1 and X2 are linked to two linkers indicated by L1 and L2, respectively, via an atom in the side chain of amino acid residues present at the same position in the human ferritin H chain contained therein.

X1 and X2 each indicate a human ferritin particle comprising one or more human ferritin H chains. Details of the human ferritin H chain and human ferritin particles are as described above. The human ferritin particles indicated by X1 and X2 may be the same or different, and are preferably the same. In a specific case, the human ferritin particle comprising one or more human ferritin H chains may comprise one or more modified human ferritin H chains and one or more unmodified human ferritin H chains.

Y indicates an IgG antibody. Details of the IgG antibody are as described above.

L1 and L2 each indicate a linker. The IgG antibody and two human ferritin particles are linked via such linkers. The linkers indicated by L1 and L2 may be the same or different, and are preferably the same. The linker may comprise a peptide moiety, but preferably does not contain a peptide moiety. This is because the peptide moiety have potential immunogenicity and are easy to hydrolyze in blood. The methods for regioselectively linking an antibody to a target substance via a linker comprising a peptide moiety are disclosed, for example, in WO2016/186206, WO2017/209471, WO2017/217347 and WO 2018/230257. The methods for regioselectively linking an antibody to a target substances via a linkers that do not contain a peptide moiety are disclosed, for example, in WO2018/199337, WO2019/240287, WO2019/240288, WO2020/009165 and WO2020/090979. According to the present invention, specific amino acid residues in a heavy chain in an antibody can be regioselectively modified without using a linker comprising a peptide moiety. The use of the linker that do not contain a peptide moiety is desirable in clinical applications.

The two human ferritin particles indicated by X1 and X2 are linked to two linkers indicated by L1 and L2, respectively, via an atom in the side chain of amino acid residues present at the same position in the human ferritin H chain contained therein. By linking the human ferritin particles to the linker via the atom in the side chain of such amino acid residues, regioselectivity is achieved for the linking site of the human ferritin particles to the IgG antibody.

In a preferred embodiment, the atom in the side chain of the amino acid residue present at the same position in the human ferritin H chain is the sulfur atom in the side chain of cysteine residue (e.g., the cysteine residue(s) present at position 91 and/or 103 according to the reference position of a natural-type human ferritin H chain). By utilizing such a cysteine residue, the number and site (regioselectivity) of the human ferritin particles to be linked to the IgG antibody can be controled highly and easily.

In a specific embodiment, the IgG antibody indicated by Y is linked to two linkers indicated by L1 and L2 via an atom in the side chain of amino acid residues present at the same position in the two heavy chain constant regions. By linking the IgG antibody to the linkers via the atom in the side chain of such amino acid residues, regioselectivity is achieved for the linking site of the IgG antibody to the human ferritin particles. As such amino acid residues, amino acid residues having a side chain which is easily modified (e.g., amino group, carboxy group, amide group, hydroxy group, thiol group) (e.g., lysine residue, aspartic acid residue, glutamic acid residue, asparagine residue, glutamine residue, threonine residue, serine residue, tyrosine residue, cysteine residue) can be utilized. Preferably, such an amino acid residue may be a lysine residue having a side chain containing an amino group (via a nitrogen atom), a tyrosine residue, a serine residue and a threonine residue having a side chain containing a hydroxy group (via an oxygen atom), or a cysteine residue having a side chain containing a thiol group (via a sulfur atom).

In a preferred embodiment, the atom in the side chain of the amino acid residues present at the same position in the two heavy chain constant regions is the nitrogen atom in the side chain of lysine residue (e.g., the lysine residue present at position 246/248, 288/290, or 317 of the IgG antibody heavy chain according to EU numbering). By utilizing such a lysine residue, the number and site (regioselectivity) of the IgG antibodies to be linked to the human ferritin particles can be controled highly and easily.

In a specific embodiment, the human ferritin particle is a particle comprising, per particle, each human ferritin H chain of the following (a) and (b) in the following average number:
(a) the number of the modified human ferritin H chains which are not linked to the linker and which are introduced with a modifying group is n; and
(b) the number of the unmodified human ferritin H chains is m;
   where n is a number from 0 to 23, m is a number from 0 to 23, and the sum of n and m is 23.

In (a) and (b) above, the sum of n and m is 23 because one of the 24 human ferritin H chains that constitute the human ferritin particle is utilized for linking the IgG antibody. That is, since the number of human ferritin H chains in the human ferritin particle not utilized for linking the IgG antibody is 23, the sum of n and m is 23.

In (a) and (b) above, n and m can be determined as follows: First, after treating a solution containing the IgG antibody of the present invention under conditions capable of dissociating the human ferritin particles, the solution is subjected to chromatography to separate the peaks of (a) the modified human ferritin H chain which is not linked to the linker and which is introduced with the modifying group, (b) the unmodified human ferritin H chain, and (c) the conjugate of two modified ferritin H chains and the IgG antibody linked via the linker. The average number of n and m can then be determined by calculating the ratio of (a) and (b) from the area ratio of the peaks of (a) and (b) and multiplying this ratio by 23. N and m may be expressed as integers, but may be expressed as numbers having values below the decimal point 1. This is because the proportions of the modified human ferritin H chain and unmodified human ferritin H chain contained in human ferritin particles linked to the IgG antibody may not be uniform in all human ferritin particles, and the averaging may calculate values below the decimal point 1.

In a specific embodiment, the human ferritin particle may be a homo 24-mer of the human ferritin H chain comprising one or more non-modified human ferritin H chains on average (mosaic form). In this case, in (a) and (b) above, n is a number from 0 to 22, m is a number from 1 to 22, and the sum of n and m is 23.

In a specific embodiment, the human ferritin particle may be a homo 24-mer of the human ferritin H chain comprising 12 or more unmodified human ferritin H chains on average (mosaic form). In this case, in (a) and (b) above, n is a number from 0 to 11, m is a number from 12 to 23, and the sum of n and m is 23. In this case, it is possible to provide human ferritin particles in which half or more of the human ferritin H chains constituting the human ferritin particles become unmodified human ferritin H chains and have a high degree of non-modification. In terms of providing human ferritin particles having a high degree of non-modification, the following (i) to (xi) are also preferred:
(i) n is a number from 0 to 10, and m is a number from 13 to 23;
(ii) n is a number from 0 to 9, and m is a number from 14 to 23;
(iii) n is a number of 0 to 8 and m is a number of 15 to 23;
(iv) n is a number of 0 to 7 and m is a number of 16 to 23;
(v) n is a number from 0 to 6, and m is a number from 17 to 23;
(vi) n is a number of 0 to 5 and m is a number of 18 to 23;
(vii) n is a number from 0 to 4, and m is a number from 19 to 23;
(viii) n is a number from 0 to 3, and m is a number from 20 to 23;
(ix) n is a number from 0 to 2, m is a number from 21 to 23;
(x) n is a number from 0 to 1, m is a number from 22 to 23;
(xi) n is an integer of 0 and m is an integer of 23.

The present invention also provides a method for preparing the complex or salt thereof of the invention.

### (I) Method (1)

The present invention provides a method for preparing a complex or salt thereof comprising (A) one IgG antibody and (B) two human ferritin particles comprising one or more human ferritin H chains linked thereto. The method of the present invention comprises the following (1)-(3):
(1) introducing first reactive groups into an IgG antibody to form a modified IgG antibody comprising two first reactive groups;
(2) introducing a second reactive group capable of reacting with the first reactive group into human ferritin particles comprising one or more human ferritin H chains to form modified particles comprising one or more modified human ferritin H chains comprising the second reactive group; and
(3) reacting the modified IgG antibody with the modified particles to obtain the complex.

The step (1) can be carried out by any method which can introduce two first reactive groups into an IgG antibody. Preferably, the introduction of the first reactive group into the IgG antibody can be performed in a regioselective manner. Regioselective introduction of IgG antibody can be performed in a manner similar to the method for regioselectively linking an antibody to a target substance as described above (see, the above references). According to the present invention, a first reactive group can be regioselectively introduced into a specific amino acid residue in two heavy chains in an IgG antibody without the use of a linker comprising a peptide portion. Specific amino acid residues include the amino acid residues described above, and lysine residues (e.g., lysine residue at position 246/248, lysine residue at position 288/290, and lysine residue at position 317) are preferred.

The step (2) can be carried out by any method which can introduce a second reactive group capable of reacting with the first reactive group into human ferritin particles comprising one or more human ferritin H chains. The introduction of the second reactive group into the human ferritin particles can be carried out via the human ferritin H chain. Preferably, the introduction of the second reactive group into the human ferritin particles can be carried out via a sulfur atom in the side chain of a cysteine residue (e.g., cysteine residues present at position 91 and/or position 103 according to the reference position of the natural-type human ferritin H chain) present in the human ferritin H chain. Such cysteine residues can be utilized to highly and easily control the number and linking site (regioselectivity) of human ferritin particles to be linked to the IgG antibody.

When a second reactive group is introduced into the human ferritin particle via a sulfur atom in the side chain of the cysteine residue present in the human ferritin H chain, the use of a thiol modifying reagent is preferred. The thiol modifying reagent used in the present invention is a reactive substance comprising at least one reaction site to a thiol group, and has an ability to add the modifying group comprising a reactive group to human ferritin. Any reagent capable of catalyzing such reactions can be used as the thiol-modifying reagent. In view of increased reaction efficiency, the thiol modifying reagent preferably comprises one or more partial structures and second reactive groups selected from the group consisting of a maleimide moiety, a benzyl halide moiety, α-haloamide moiety, α-haloketone moiety, alkene moiety, alkyne moiety, fluoroaryl moiety, nitroaryl moiety, methylsulfonyloxadiazole moiety, and disulfide moiety.

In step (3), the modified IgG antibody obtained in step (1) is reacted with the modified particles obtained in step (2) described above. Thereby, the first reactive group introduced into the modified IgG antibody and the second reactive group introduced into the modified particle can react with each other to obtain the complex or salt thereof of the invention. The molar ratio of the modified IgG antibody to the modified particles in the reaction may be in the range of, for example, 30:1 to 1:30, preferably 20:1 to 1:20, more preferably 15:1 to 1:15. Alternatively, the reaction may be set based on the molar ratio of the first reactive group introduced to the modified IgG antibody to the second reactive group introduced into the modified particle. The molar ratio of the first reactive group to the second reactive group in the reaction may be in the range of, for example, 1:1 to 1:30, preferably 1:1 to 1:20, more preferably 1:1 to 1:15.

The reaction of steps (1) to (3) above can be performed as appropriate under conditions that cannot cause denaturation or degradation of ferritin (e.g., cleavage of amide bonds) (mild conditions). For example, such a reaction may be performed in a suitable reaction system such as a buffer under temperature conditions that may not cause denaturation or degradation of ferritin (for example, about 15-60°C, preferably 15-45°C). The pH of the buffer is, for example, 3.0 to 12.0, preferably 5.0 to 9.0, more preferably 6.0 to 8.0. The buffer may comprise a component such as a catalyst. The reaction time is, for example, from 1 minute to 20 hours, preferably from 10 minutes to 15 hours, more preferably from 20 minutes to 10 hours, even more preferably from 30 minutes to 8 hours.

### (II) Methods (2-1) and (2-2)

In a specific embodiment, the method of the present invention may be a method for producing a complex or salt thereof comprising (A) one IgG antibody and (B) two human ferritin particles comprising one or more modified human ferritin H chains and one or more unmodified human ferritin H chains linked thereto.

In one embodiment, the method (2-1) of the present invention includes the following (1)-(4):
(1) introducing first reactive groups into an IgG antibody to form a modified IgG antibody comprising two first reactive groups;
(2) introducing a second reactive group capable of reacting with the first reactive group into human ferritin particles comprising two or more human ferritin H chains to form first modified particles comprising two or more modified human ferritin H chains comprising the second reactive group;
(3) replacing a portion of the two or more modified human ferritin H chains in the first modified particles with unmodified human ferritin H chains to form second modified particles comprising one or more modified human ferritin H chains and one or more unmodified human ferritin H chains; and
(4) reacting the modified IgG antibody with the second modified particles to obtain the complex.

The steps (1) and (2) in the method (2-1) can be performed in a manner similar to the steps (1) and (2) in the method (1).

In the step (3) in the method (2-1), in a first modified particle comprising two or more modified human ferritin H chains comprising a second reactive group, a portion of the two or more modified human ferritin H chains is replaced with the unmodified human ferritin H chain. In this step, a portion of the two or more modified human ferritin H chains (i.e., at least one modified human ferritin H chain) is replaced with the unmodified human ferritin H chain to form a second modified particle comprising one or more modified human ferritin H chains and one or more unmodified human ferritin H chains.

The above replacement can be carried out by coexistence of the first modified particle comprising two or more modified human ferritin H chains comprising a second reactive group and the unmodified human ferritin particle comprising the unmodified human ferritin H chain under conditions capable of dissociating human ferritin H chains from human ferritin particles and reassociating human ferritin particles from human ferritin H chains. Such dissociation and reassociation have been utilized in encapsulating various substances in an internal cavity of ferritin particles (e.g., WO 2020/090708; Chinese Patent Application Publication 106110333; Journal of Controlled Release 196(2014) 184-196; Biomacromics 2016, 17, 514-522; Proc Natl Acad Sci USA. 2014 111(41): 14900-5). Thus, in this step it may be performed simultaneously to encapsulate the substance in the internal cavity of the ferritin particle. Preferably, the condition which can achieve the dissociation is to place ferritin particles in an aqueous solution having a pH of 1.5-3.0 adjusted with an acid (e.g., hydrochloric acid, glycine hydrochloride, sulfuric acid) (e.g., 5-60 minutes at 4-30°C). The condition can achieve the reassociation is to place ferritin particles in an aqueous solution having a pH of 1.5 to 3.0 (e.g., 30-180 minutes at 4-30°C). For example, buffer (e.g., Tris buffer, HEPES buffer, phosphate buffer, borate buffer, citrate buffer, carbonic acid buffer, glycine buffer) may be added to the solution containing the dissociated product to adjust the solution to a pH of 5.0 to 10.0.

By setting the molar ratio of unmodified human ferritin particles to the first modified particles high, the proportion of unmodified human ferritin H chains in the second modified particles can be increased. The molar ratio of the unmodified human ferritin particles to the first modified particles (unmodified human ferritin particles/first modified particles) may be, for example, 1 or more, 1.5 or more, 2 or more, 2.5 or more, 3 or more, 4 or more, 5 or more, 6 or more, 7 or more, 9 or more, or 10 or more. The molar ratio may also be 50 or less, 40 or less, 30 or less, or 20 or less. If an excess amount of unmodified human ferritin particles is used relative to the first modified particles, the average number of unmodified human ferritin H chains in the second modified particles can be set to one or less. In this case, since one is utilized in the reaction of step (4), modified human ferritin particles rich in unmodified human ferritin H chain can be produced, including one modified human ferritin H chain which can be linked with the linker in response to the reaction of step (4), and 23 non-modified human ferritin H chains.

The step (4) in the method (2-1) can be performed in a similar manner to the step (3) in the method (1).

In another embodiment, the method (2-2) of the present invention includes the following (1)-(4):
(1) introducing first reactive groups into an IgG antibody to form an IgG antibody comprising two first reactive groups;
(2) introducing a second reactive group capable of reacting with the first reactive group into human ferritin particles comprising two or more human ferritin H chains to form first modified particles comprising two or more modified human ferritin H chains comprising the second reactive group;
(3) reacting the modified IgG antibody with the first modified particles to form a complex or salt thereof comprising (A') one IgG antibody and (B') two human ferritin particles comprising two or more modified human ferritin H chains linked thereto; and
(4) replacing a portion of the two or more modified human ferritin H chains in the complex or salt thereof comprising (A') one IgG antibody and (B') two human ferritin particles comprising two or more modified human ferritin H chains linked thereto with an unmodified human ferritin H chain to obtain the complex or salt thereof comprising (A) one IgG antibody and (B) two human ferritin particles comprising one or more modified human ferritin H chains and one or more unmodified human ferritin H chains linked thereto.

The steps (1)-(3) in the method (2-2) can be carried out in a similar manner to the steps (1)-(3) in the method (1).

In the step (4) in the method (2-2), in a complex or salt thereof comprising (A') one IgG antibody and (B') two human ferritin particles comprising two or more modified human ferritin H chains linked thereto, a portion of the two or more modified human ferritin H chains is replaced with the unmodified human ferritin H chain. In this step, a portion of the two or more modified human ferritin H chains (i.e., at least one modified human ferritin H chain) is replaced with the unmodified human ferritin H chain to form a complex or salt thereof comprising (A) one IgG antibody and (B) two human ferritin particles comprising one or more modified human ferritin H chains and one or more unmodified human ferritin H chains.

The above replacement can be carried out by coexistence of a complex or salt thereof comprising (A') one IgG antibody and (B') two human ferritin particles comprising two or more modified human ferritin H chains, and unmodified human ferritin particles comprising an unmodified human ferritin H chain under conditions capable of dissociation and reassociation as described above. Such dissociation and reassociation have been utilized in encapsulating various substances in the internal cavity of ferritin particles. Thus, in this step, it may be performed simultaneously to encapsulate the substance in the internal cavity of the ferritin particle. Preferably, the conditions which can achieve the dissociation and reassociation are as described above.

By setting the proportion of unmodified human ferritin particles to the raw material complex or salt thereof high, the proportion of unmodified human ferritin H chains in the resulting complex can be increased. The molar ratio of unmodified human ferritin particles to the raw material complex (unmodified human ferritin particle/raw complex) may be, for example, 1 or more, 1.5 or more, 2 or more, 2.5 or more, 3 or more, 4 or more, 5 or more, 6 or more, 7 or more, 9 or more, or 10 or more. The molar ratio may also be 50 or less, 40 or less, 30 or less, or 20 or less. If an excess amount of unmodified human ferritin particles are used relative to the raw material complex or salt thereof, the average number of unmodified human ferritin H chains in the resulting complex or salt thereof can be set to one. In this case, modified human ferritin particles rich in unmodified human ferritin H chain can be produced, including one modified human ferritin H chain and 23 unmodified human ferritin H chains linked to a linker.

The method may further comprise encapsulating the substance in the internal cavity of the human ferritin particle and reacting a functional substance with such a modifying group if the modified human ferritin H chain having the modifying group remains in the human ferritin particle.

Confirmation of the complex or salt thereof of the present invention can be performed, for example, by electron microscopy, electrophoresis, chromatography (e.g., gel filtration chromatography, ion exchange chromatography, reverse phase column chromatography, HPLC), and/or mass spectrometry. When the complex or salt thereof of the invention comprises a modified human ferritin H chain having a modifying group, it is selected, as appropriate, from the above method, depending on the type and molecular weight of the modifying group. Confirmation of regioselectivity can be performed, for example, by peptide mapping. Peptide mapping can be performed, for example, by protease (e.g. trypsin, chymotrypsin) treatment and mass spectrometry. Endoprotease is preferred as the protease. Such endoproteases include, for example, trypsin, chymotrypsin, Glu-C, Lys-N, Lys-C, and Asp-N. The modified ferritin can be purified by any method such as chromatography (e.g. chromatography as described above, and affinity chromatography).

### Examples

Hereinafter, the present invention will be described in more detail by Examples, but the present invention is not limited to the Examples.

Reference Example 1: Preparation of human ferritin H chain

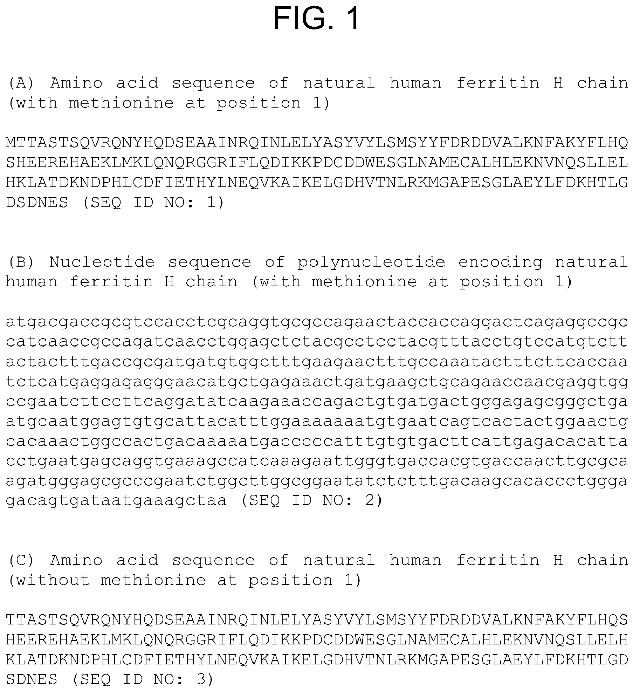

DNA (SEQ ID NO: 2) encoding a human ferritin H chain (FTH (SEQ ID NO: 1)) was totally synthesized. When an expression plasmid of this DNA is introduced into E. coli to express the human ferritin H chain in E. coli, methionine at an initiation codon becomes N-formylmethionine and is removed by post-translational modification. Therefore, the human ferritin H chain defined by the amino acid sequence of SEQ ID NO: 3 from which a methionine residue at position 1 in the amino acid sequence of SEQ ID NO: 1 has been removed is obtained (FIG. 1). Hereinafter, the position of an amino acid residue of a human ferritin is specified based on a typical natural human ferritin H chain defined by the amino acid sequence of SEQ ID NO: 1. If necessary, the position of the amino acid sequence of SEQ ID NO: 3 may also be described.

PCR was performed using synthetic DNA encoding a human ferritin H chain as a template and using 5'-GAAGGAGATATACATATGACGACCGCGTCCACCTCG-3' (SEQ ID NO: 4) and 5'-CTCGAATTCGGATCCTTAGCTTTCATTATCACTGTC-3' (SEQ ID NO: 5) as primers. In addition, PCR was performed using pET20 (Merck) as a template and using 5'-TTTCATATGTATATCTCCTTCTTAAAGTTAAAC-3' (SEQ ID NO. 6) and 5'-TTTGGATCCGAATTCGAGCTCCGTCG-3' (SEQ ID NO. 7) as primers. Each of the obtained PCR products was purified with a Wizard DNA Clean-Up System (Promega Corporation), and then subjected to In-Fusion enzyme treatment with In-Fusion HD Cloning Kit (Takara Bio Inc.) at 50°C for 15 minutes, thereby constructing an expression plasmid (pET20-FTH) on which a gene encoding FTH was mounted.

Subsequently, Escherichia coli BL21 (DE3) into which the constructed pET20-FTH was introduced was cultured in a flask containing 100 ml of LB medium (containing 10 g/l Bacto-typtone, 5 g/l Bacto-yeast extract, 5 g/l NaCl, and 100 mg/l ampicillin) at 37°C for 24 hours. The obtained bacterial cells were ultrasonically disrupted, and then the supernatant was heated at 60°C for 20 minutes. After heating, the supernatant obtained by cooling and centrifugation was injected into a HiPerp Q HP column (Cytiva) equilibrated with a 50 mM tris-HCl buffer (pH 8.0), and subjected to a salt concentration gradient with a 50 mM tris-HCl buffer (pH 8.0) containing 0 mM to 500 mM NaCl, thereby separating and purifying a target protein due to a difference in surface charge. A solvent of a solution containing the protein was replaced with a 10 mM tris-HCl buffer (pH 8.0) by centrifugal ultrafiltration using Vivaspn 20-100K (Cytiva). The solution was injected into a HiPrep 26/60 Sephacryl S-300 HR column (Cytiva) equilibrated with a 10 mM tris-HCl buffer (pH 8.0), and FTH was separated and purified. A solution containing the FTH was concentrated by centrifugal ultrafiltration using Vivaspn 20-100K (Cytiva), and the concentration of a protein contained was determined by a protein assay CBB solution (Nacalai Tesque, Inc.) using bovine albumin as a standard. As a result, 1 ml of a solution containing 5 mg/ml FTH could be obtained per 100 ml of the culture solution.

Reference Example 2: Specific modification of human ferritin H chain with ethyl maleimide and ESI-TOFMS analysis

### (2-1) Specific modification of human ferritin H chain with ethyl maleimide and ESI-TOFMS analysis

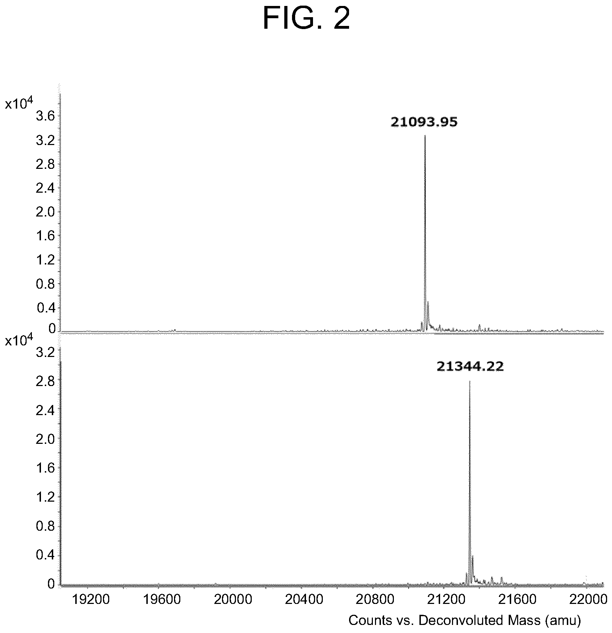

The human ferritin H chain expressed in Reference Example 1 was dissolved in a pH 7.4 PBS buffer so as to have a concentration of 5.0 mg/ml. Ethyl maleimide was dissolved in N,N-dimethylformamide so as to have a concentration of 20 mM. Six equivalents (based on a molar amount, the same applies hereinafter) of ethyl maleimide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material, and a peak at 21344 at which two molecules of ethyl maleimide (molecular weight: 125) were introduced was confirmed for the product (FIG. 2) .

### Reference Example 3: Peptide mapping of ethyl maleimide-introduced product of human ferritin H chain (3-1) Trypsin treatment of ethyl maleimide-introduced product of human ferritin H chain

To a 1.5 mL low-adsorption micro test tube, 10 µL of a sample solution (containing the ethyl maleimide-introduced product of a human ferritin heavy chain prepared in Reference Example 2), 30 µL of a 50 mM tris-hydrochloric acid buffer/8M urea, and 10 µL of trifluoroethanol were added, and the resulting mixture was stirred. Thereafter, 15 µL of a 48 mM dithiothreitol aqueous solution was added thereto, and the resulting mixture was heated at 65°C for one hour. Thereafter, 15 µL of a 120 mM iodoacetamide aqueous solution was added thereto, and the resulting mixture was caused to react in a dark place at room temperature for 60 minutes. After the reaction, 5 µL of a 48 mM dithiothreitol aqueous solution was added to the solution to suppress overreaction. 150 µL of a 50 mM tris-hydrochloric acid buffer was added thereto, and the resulting mixture was stirred. 2.5 µL of a 100 ng/ µL trypsin aqueous solution (Proteomics Grade, Code No. T6567-5 × 20 µg (SIGMA)) was added thereto, and the resulting mixture was allowed to stand at 37°C for one hour. Furthermore, 2.5 µL of a 100 ng/µL trypsin aqueous solution was added thereto, and the resulting mixture was subjected to enzymatic digestion at 37°C for 15 hours. After the digestion, 15 µL of a 20% trifluoroacetic acid aqueous solution was added to the solution to stop the reaction, and the resulting mixture was diluted 10 times with a 0.1% trifluoroacetic acid aqueous solution and then subjected to LC-MS/MS measurement.

### (3-2) Conditions for LC-MS/MS measurement of human ferritin heavy chain

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 1 µL
Gradient condition (B%): 2% (0.0 minute to 0.5 minute), 2% → 30% (0.5 minute to 23.5 minutes), 30% → 75% (23.5 minutes to 25.5 minutes), and 75% (25.5 minutes to 35.0 minutes).

### (Mass Spectrometer Analysis Conditions)

Ionization: ESI, Positive mode
Scan type: Data Dependent Aquisition
Activation Type: Collision Induced Dissociation(CID)
Data acquisition was performed using Xcalibur 3.0 (Thermo Fisher Scientific) and Thermo Orbitrap Fusion Tune Application 2.0 (Thermo Fisher Scientific) as accompanying software.

### (3-3) Analysis condition of modified site of human ferritin H chain

Modified site analysis of an LC-MS/MS measurement result was performed using BioPharma Finder 3.0 (Thermo Fisher Scientific).

The analysis with BioPharma Finder was performed by setting S/N threshold to 20 and setting MS Noise Level to 1000. A digestive enzyme was set to Trypsin, and Specificity was set to High. Furthermore, the Mass Accuracy at the time of peptide identification was set to 5 ppm. For Dynamic Modifications, oxidation of methionine (+15.995 Da),a modified product to a cysteine residue (N-ethyl maleimide-introduced product (+125.048Da), and carbamidomethylation with iodoacetamide (+57.021Da)) were set. In addition, a filter was set such that only those with a Confidence Score of 80 or higher and with observed MS/MS were obtained.

In addition, the amino acid sequence indicated in SEQ ID NO: 1 was used as data of an amino acid sequence to be searched for a modified site.

### (3-4) Analysis result of modified site of human ferritin H chain by LC-MS/MS

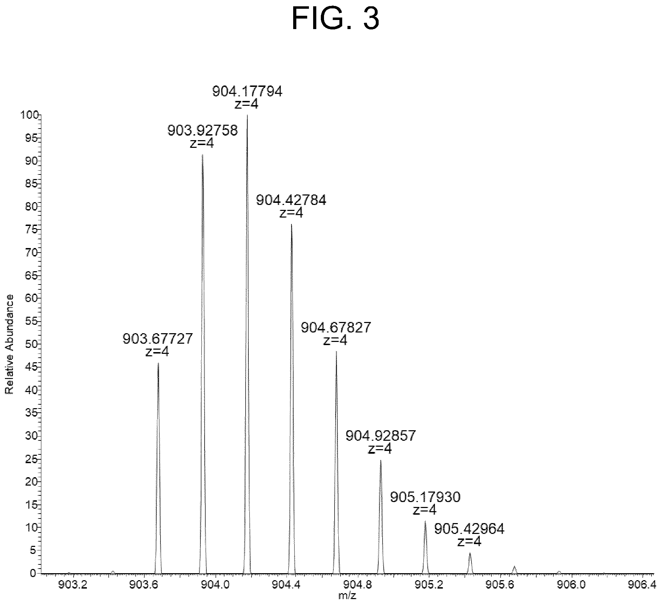

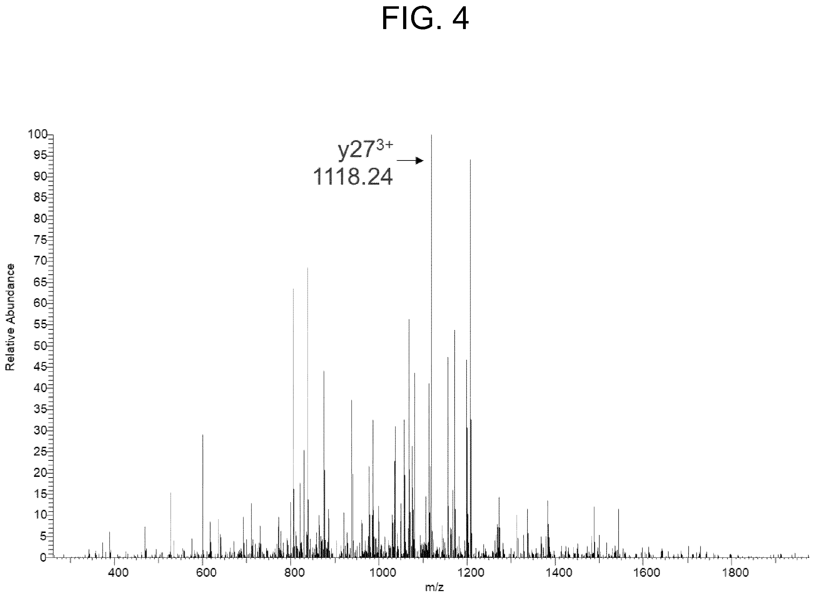

As a result of analysis using LC-MS/MS, an MS spectrum of a peptide fragment of IFLQDIKKPDCDDWESGLNAMECALHLEK (SEQ ID NO: 8), which is a peptide consisting of 29 amino acid residues containing a modified site (N-ethyl maleimide-introduced product (+125.048Da)) to two cysteine residues generated by trypsin digestion of a specific modified product of a human ferritin heavy chain by ethyl maleimide of Reference Example 2 (measured value: m/z 903.67727, theoretical value: 903.67738, tetravalent) was observed (FIG. 3), and from a CID spectrum, a product ion of m/z 1118.24 (theoretical value: 1117.85) corresponding to trivalent y27 indicating modification of cysteine residues at positions 91 and 103 of a human ferritin H chain (at positions 90 and 102 of the amino acid sequence of SEQ ID NO: 3) was confirmed (FIG. 4).

### Reference Example 4: Specific modification of human ferritin H chain with p-azidophenacyl bromide and ESI-TOFMS analysis

### (4-1) Specific modification of human ferritin H chain by six equivalents of p-azidophenacyl bromide and ESI-TOFMS analysis

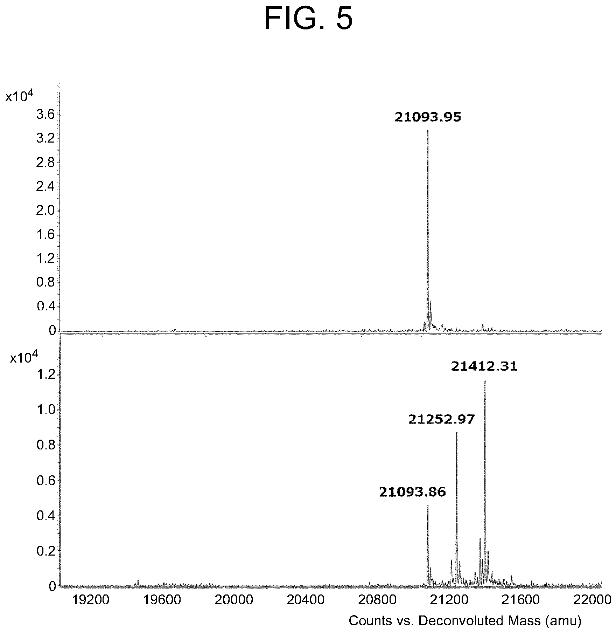

The human ferritin H chain expressed in Reference Example 1 was dissolved in a pH 7.4 PBS buffer so as to have a concentration of 5.0 mg/ml. p-Azidophenacyl bromide was dissolved in N,N-dimethylformamide so as to have a concentration of 20 mM. Six equivalents of p-azidophenacyl bromide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material. The peak of the raw material, a peak at 21252 at which one molecule of p-azidophenacyl (molecular weight: 160) was introduced, and a peak at 21412 at which two molecules of p-azidophenacyl were introduced were confirmed for the product (FIG. 5).

### (4-2) Specific modification of human ferritin H chain by 12 equivalents of p-azidophenacyl bromide and ESI-TOFMS analysis

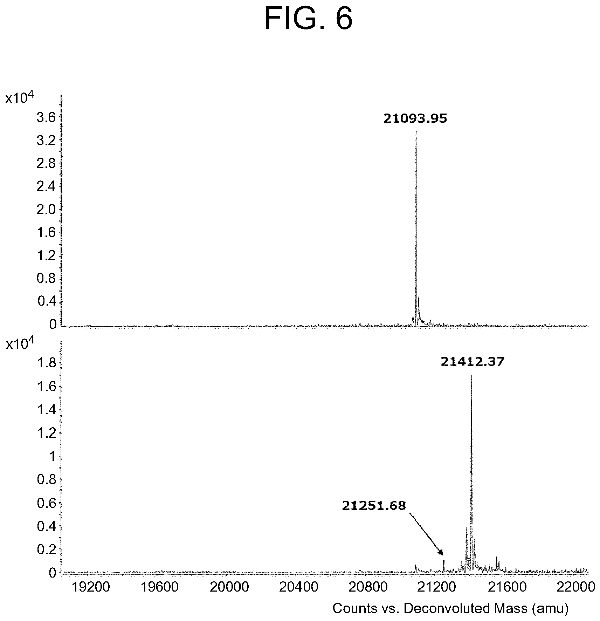

The human ferritin H chain expressed in Reference Example 1 was dissolved in a pH 7.4 PBS buffer so as to have a concentration of 5.0 mg/ml. p-Azidophenacyl bromide was dissolved in N,N-dimethylformamide so as to have a concentration of 20 mM. 12 equivalents of p-azidophenacyl bromide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material. A peak at 21252 at which one molecule of p-azidophenacyl (molecular weight: 160) was introduced, and a peak at 21412 at which two molecules of p-azidophenacyl were introduced were confirmed for the product (FIG. 6).

### Reference Example 5: Peptide mapping of p-azidophenacyl-introduced product of human ferritin H chain (5-1) Trypsin treatment of p-azidophenacyl-introduced product of human ferritin H chain

A treatment similar to Reference Example 3 (3-1) was performed except that the sample solution containing the p-azidophenacyl-introduced product of the human ferritin H chain prepared in Reference Example 4 (4-2) was used instead of the sample solution containing the ethyl maleimide-introduced product of the human ferritin heavy chain prepared in Reference Example 2, and the ethyl maleimide-introduced product of the human ferritin heavy chain was subjected to LC-MS/MS measurement.

### (5-2) Conditions for LC-MS/MS measurement of human ferritin H chain

An analyzer, HPLC analysis conditions, and mass spectrometer analysis conditions are the same as those in Reference Example 3 (3-2).

### (5-3) Analysis conditions of modified site of human ferritin H chain

Modified site analysis of an LC-MS/MS measurement result was performed using BioPharma Finder 3.0 (Thermo Fisher Scientific).

The analysis with BioPharma Finder was performed by setting S/N threshold to 20 and setting MS Noise Level to 1000. A digestive enzyme was set to Trypsin, and Specificity was set to High. Furthermore, the Mass Accuracy at the time of peptide identification was set to 5 ppm. For Dynamic Modifications, oxidation of methionine (+15.995 Da), a modified product to a cysteine residue (p-azidophenacyl-introduced product (+159.043262Da), a p-aminophenacyl-introduced product (+133.052764Da), and carbamidomethylation with iodoacetamide (+57.021Da)) were set. In addition, a filter was set such that only those with a Confidence Score of 80 or higher and with observed MS/MS were obtained.

In addition, the amino acid sequence indicated in SEQ ID NO: 1 was used as data of an amino acid sequence to be searched for a modified site.

Note that since reductive alkylation with dithiothreitol and iodoacetamide is performed during digestion, the p-azidophenacyl-introduced product is partially reduced to become a p-aminophenacyl-introduced product.

### (5-4) Analysis result of modified site of human ferritin H chain by LC-MS/MS

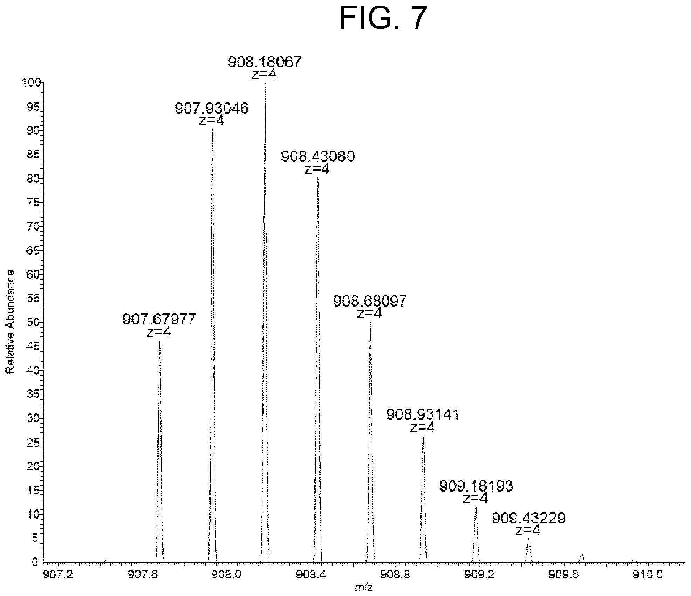

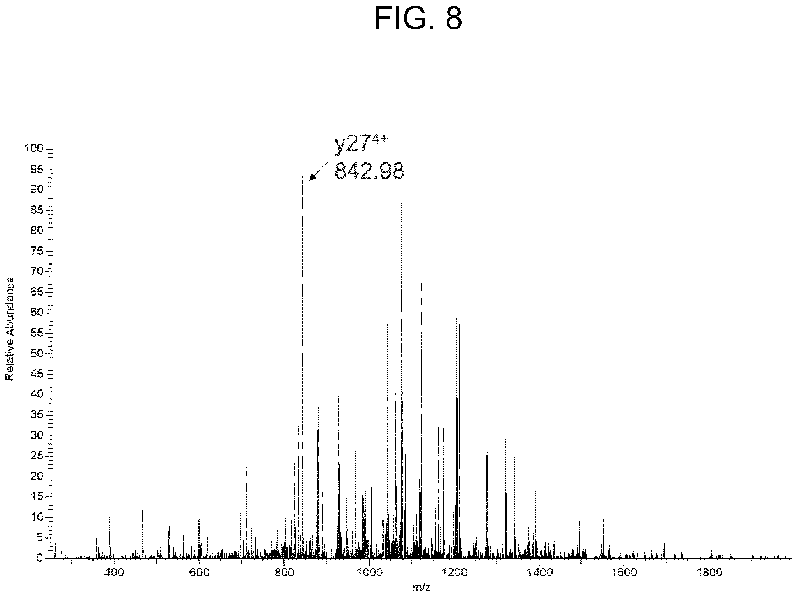

As a result of analysis using LC-MS/MS, an MS spectrum of a peptide fragment of IFLQDIKKPDCDDWESGLNAMECALHLEK (SEQ ID NO: 8), which is a peptide consisting of 29 amino acid residues containing a modified site (p-aminophenacyl-introduced product (+133.052764Da) reduced by dithiothreitol) to two cysteine residues generated by trypsin digestion of a specific modified product of a human ferritin heavy chain by p-azidophenacyl of Reference Example 4 (4-2) (measured value: m/z 907.67977, theoretical value: 907.67992, tetravalent) was observed (FIG. 7), and from a CID spectrum, a product ion of m/z 842.98 (theoretical value: 842.64) corresponding to tetravalent y27 indicating modification of cysteine residues at positions 91 and 103 of a human ferritin H chain (at positions 90 and 102 of the amino acid sequence of SEQ ID NO: 3) was confirmed (FIG. 8).

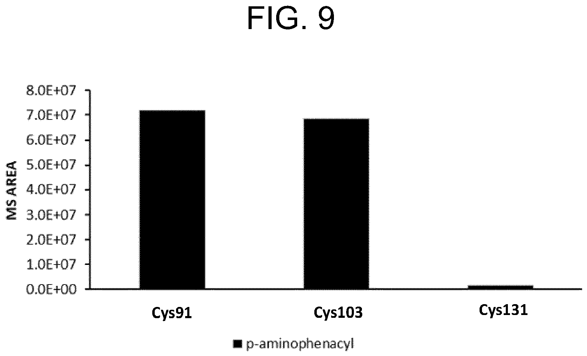

Based on the analysis result obtained in (5-4), peak area values at positions 91, 103, and 131 (at positions 90, 102, and 130 in the amino acid sequence of SEQ ID NO: 3) of the human ferritin H chain were compared (FIG. 9). It can be seen that positions 91 and 103 of the human ferritin H chain (positions 90 and 103 in the amino acid sequence of SEQ ID NO: 3) are selectively modified.

### Reference Example 6: Specific modification of human ferritin H chain with Cy5-PEG-maleimide and ESI-TOFMS analysis

### (6-1) Specific modification of human ferritin H chain with Cy5-PEG-maleimide and ESI-TOFMS analysis

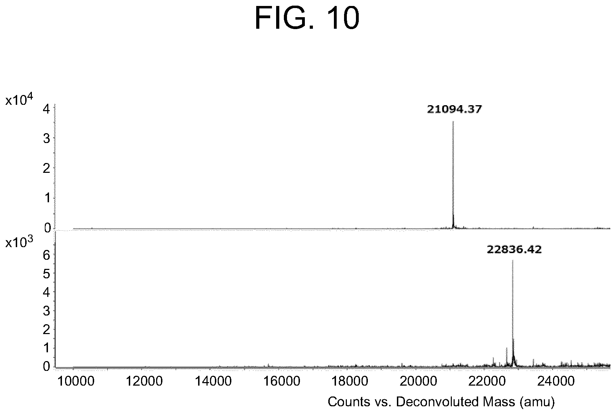

The human ferritin H chain expressed in Reference Example 1 was dissolved in a pH 7.4 PBS buffer so as to have a concentration of 5.0 mg/ml. Cy5-PEG-maleimide (Broad Pharm, BP-23037) was dissolved in N,N-dimethylformamide so as to have a concentration of 20 mM. Six equivalents of Cy5-PEG-maleimide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human ferritin H chain as a raw material. The peak of the raw material and a peak at 22836 at which two molecules of Cy5-PEG-maleimide (molecular weight: 871) were introduced were confirmed for the product (FIG. 10).

### Reference Example 7: Specific modification of human ferritin H chain with DBCO-PEG4-maleimide and ESI-TOFMS analysis

### (7-1) Specific modification of human ferritin H chain with DBCO-PEG4-maleimide and ESI-TOFMS analysis

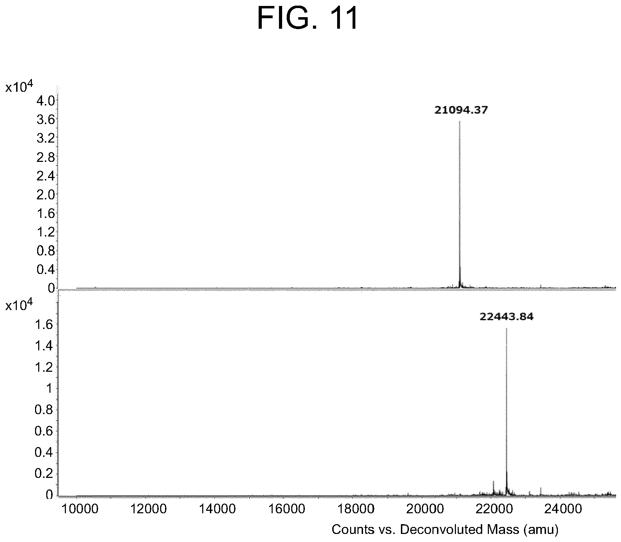

The human ferritin H chain expressed in Reference Example 1 was dissolved in a pH 7.4 PBS buffer so as to have a concentration of 5.0 mg/ml. DBCO-PEG4-maleimide (Broad Pharm, BP-22294) was dissolved in N,N-dimethylformamide so as to have a concentration of 20 mM. Six equivalents of DBCO-PEG4-maleimide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human ferritin H chain as a raw material. The peak of the raw material and a peak at 22443 at which two molecules of DBCO-PEG4-maleimide (molecular weight: 673) were introduced were confirmed for the product (FIG. 11).

### Reference Example 8: Specific modification of human ferritin H chain with 1,3-dichloroacetone, oxime ligation with aminooxy-PEG5-azide, and ESI-TOFMS analysis (8-1) Specific modification of human ferritin H chain with 1,3-dichloroacetone and ESI-TOFMS analysis

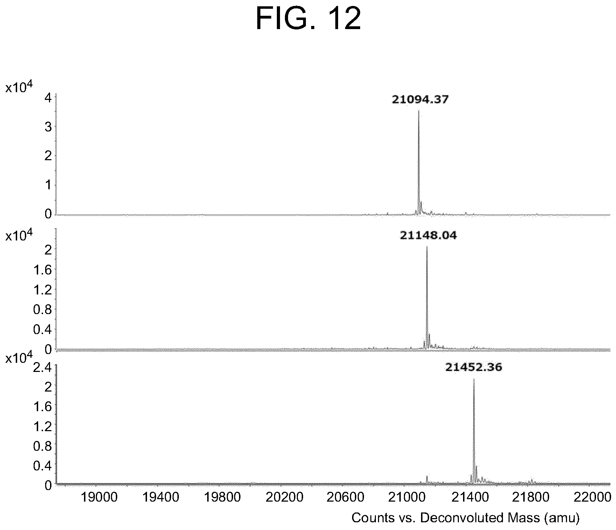

The human ferritin H chain expressed in Reference Example 1 was dissolved in a pH 7.4 PBS buffer so as to have a concentration of 5.0 mg/ml. 1,3-Dichloroacetone was dissolved in N,N-dimethylformamide so as to have a concentration of 20 mM. Six equivalents of 1,3-dichloroacetone was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human ferritin H chain as a raw material, and a peak at 21148 at which one molecule of acetone (molecular weight: 54) was introduced for the product was confirmed (FIG. 12).

### (8-2) Specific modification of oxime ligation with aminooxy-PEG5-azide and ESI-TOFMS analysis

The human ferritin H chain acetone modified product obtained in (8-1) was dissolved in a pH 4.7 acetic acid buffer so as to have a concentration of 2.0 mg/ml. Aminooxy-PEG5-azide (Broad Pharm, BP-23194) was dissolved in N,N-dimethylformamide so as to have a concentration of 20 mM. Ten equivalents of aminooxy-PEG5-azide was added to the aqueous solution containing the human ferritin H chain acetone modified product. The resulting mixture was stirred, and then subjected to a shaking reaction at 37°C for 16 hours. The reaction solution was replaced with a 20 mM ammonium acetate buffer, and the mass was measured by ESI-TOFMS. A peak was observed at 21148 for the human ferritin H chain acetone modified product as a raw material. A peak at 21452 at which one molecule of aminooxy-PEG5-azide (molecular weight: 304) was introduced was confirmed for the product (FIG. 12).

### Example 1: Synthesis of specifically modified antibodies

### (1-1) Synthesis of regiospecifically modified antibody by DBCO-maleimide

In this Example, a thiol group-introduced trastuzumab was reacted with dibenzocyclooctyne (DBCO)-maleimide.

As an antibody, the antibody derivative (thiol group-introduced trastuzumab) described in Example 81-7 of WO2019/240287 was used. This antibody derivative has the following structure in which thiol groups are regioselectively introduced into trastuzumab (humanized IgG1 antibody) via the amino group of the side chain of the lysine residue at position 246 or 248 of the antibody heavy chain (the position of the lysine residue follows EU numbering): 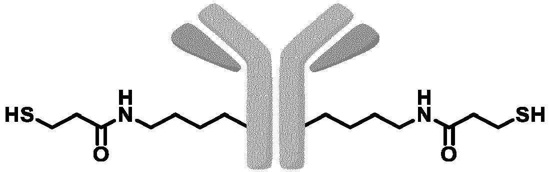 (In the above structure, NH-CH₂-CH₂-CH₂-CH₂- extending from the antibody heavy chain corresponds to the side chain of the lysine residue, and the thiol-containing group HS-CH₂-CH₂-C(=O) is added to the amino group in the side chain of the lysine residue.)

As the DBCO-maleimide, the following was used.

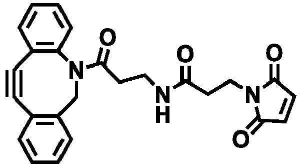

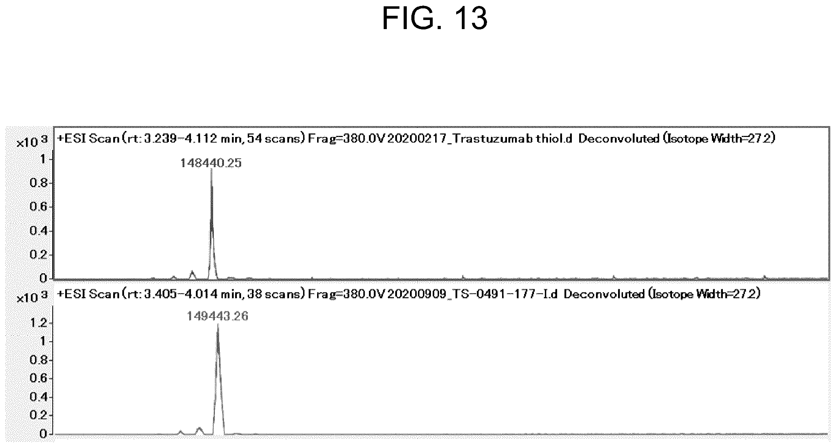

To a solution of the above-mentioned antibody derivative (2 mg/mL) in a buffer (PBS buffer at pH 7.4), a solution of 10 equivalents of dibenzocyclooctyne (DBCO)-maleimide in DMF (10 mM) was added, and after shaking at 25°C for 2 hours, the reactant was purified using NAP-5 Columns (GE Healthcare). The reaction solution was replaced with 20 mM ammonium acetate buffer, and the mass was measured by ESI-TOFMS under non-reducing conditions according to the procedures previously reported (Anal. Chem., 2019, 91, 20, 12724-12732). As a result, a peak at 149443 in which two DBCO groups were introduced into the antibody was confirmed (FIG. 13).

Therefore, by reacting two thiol groups in the antibody derivative with the maleimide group in DBCO-maleimide, it was confirmed that a DBCO modified antibody in which two DBCO groups were introduced was produced.

### (1-2) Synthesis of regiospecifically modified antibody by DBCO-PEG-maleimide

In this Example, the thiol group-introduced trastuzumab was reacted with dibenzocyclooctyne (DBCO)-PEG-maleimide).

As an antibody, the above antibody derivative described in Example (1-1) was used.

As the DBCO-PEG-maleimide, the following was used.

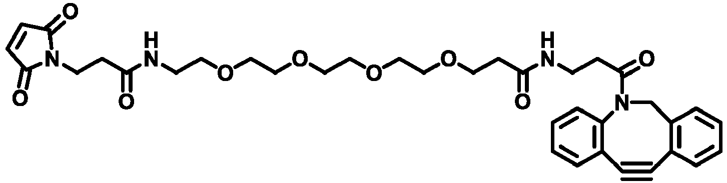

To a solution of the above antibody derivative (2 mg/mL) in a buffer (PBS buffer at pH 7.4), a solution of 10 equivalents of dibenzocyclooctane-PEG4-maleimide in DMF (10 mM) was added, and after shaking reaction at 25°C for 2 hours, the reactant was purified using NAP-5 Columns (GE Healthcare). The reaction solution was replaced with 20 mM ammonium acetate buffer and mass was measured by ESI-TOFMS under non-reducing conditions according to procedures previously reported (Anal. Chem., 2019, 91, 20, 12724-12732).

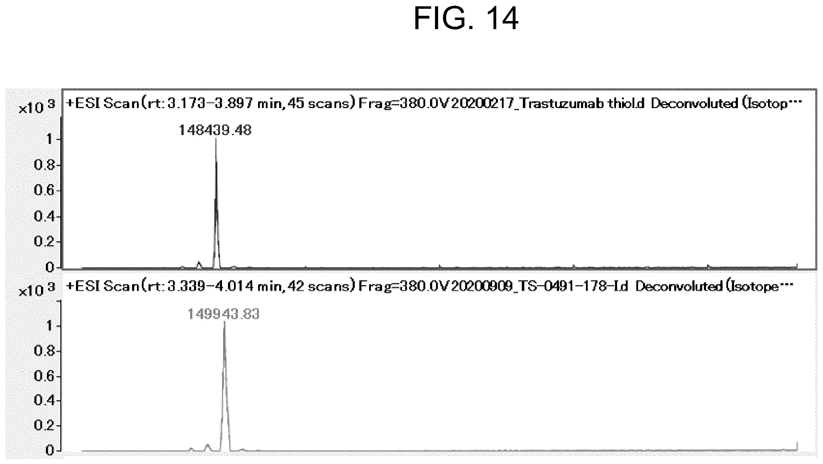

As a result, a peak at 149944 in which two DBCO groups were introduced into the antibody was confirmed (FIG. 14). Therefore, by reacting two thiol groups in the antibody derivative with the maleimide group in the DBCO-PEG-maleimide, it was confirmed that a DBCO-PEG modified antibody in which two DBCO-PEG groups were introduced was produced.

### Example 2: Specific modification of human ferritin H chains by p-azidophenacyl bromide

In this Example, human ferritin particles consisting of 24-mer of human ferritin H chains were reacted with p-azidophenacyl bromide.

Human ferritin-particles were dissolved in PBS buffer at pH 7.4 and the concentration was set to 2.5 mg/ml (final concentration: 5 µM). p-azidophenacyl bromide was dissolved in N,N-dimethylformamide so as to be 100 mM. 106 equivalents of p-azidophenacyl bromide (final concentration: 528 µM) was added to an aqueous solution containing human ferritin particles. After stirring, the solution was shaken at 25°C for 1 hour. The reaction solution was replaced with 20 mM ammonium acetate buffer and mass was measured by ESI-TOFMS.

As a result, a peak was observed at 21094 as a monomer for the human ferritin H chain which constitutes the human ferritin particle. For the product, a peak at 21412 was confirmed which is a monomer in which two p-azidophenacyl (molecular weight: 160) were introduced into the human ferritin H chain.

Therefore, it was confirmed that a ferritin azide having 48 azide groups per cage structure consisting of 24-mer of a human ferritin H chain (in consideration of the above reference example, 24-mer of the modified human ferritin H chain in which a modifying group containing an azide group was introduced into the sulfur atom in the side chain of the two cysteine residues at positions 91 and 103 in the human ferritin H chain) was produced.

### Example 3: Construction of antibody-human ferritin Complex (1)

In this example, the DBCO modified antibody obtained in Example 1 was reacted with the ferritin azide obtained in Example 2. The alkyne moiety in DBCO can readily react with the azide group by click reaction under mild conditions. Thus, by reacting the DBCO modified antibody with the ferritin azide, an IgG antibody linked to two human ferritin particles (antibody-human ferritin complex) can be produced.

The DBCO modified antibody obtained in Example 1 and the ferritin azide particle obtained in Example 2 were dissolved in PBS buffer at pH 7.4 so as to be a final concentration of 2.5 µM, and then shaken at 25°C for 1 hour. The resulting sample was diluted 12.5 times with water, mixed at 1:1 with Laemmli sample buffer (Biorad) containing 5% β-mercaptoethanol, and then heat-treated at 95°C for 5 minutes. A 10 µl aliquot of the solutions was analyzed by SDS-PAGE.

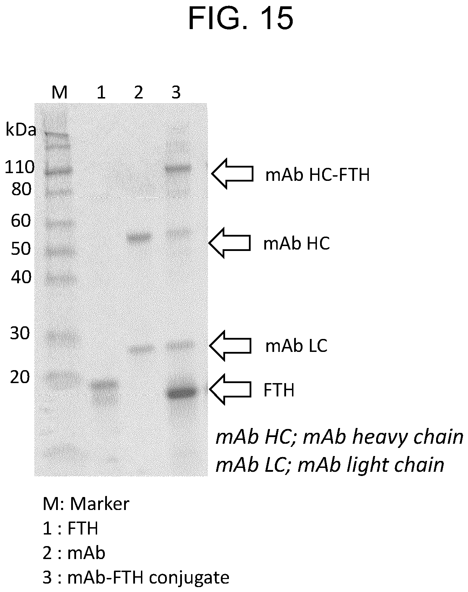

As a result, a protein band around 100 kDa in which the ferritin H chain and the antibody heavy chain were conjugated was observed only in the conjugated sample (FIG. 15), which was not observed by the antibody or the ferritin H chain (monomer) alone. Since the molecular weight of the ferritin azide H chain (monomer) is 21 kDa and the molecular weight of the antibody heavy chain is from 50 kDa to 60 kDa, it is considered that the band obtained by the conjugation reaction is a protein in which two ferritin azide H chains (monomers) are covalently bonded to the antibody heavy chain.

### Example 4: TEM analysis of antibody-human ferritin complex

The antibody-human ferritin complex obtained in Example 3 was confirmed by transmission electron-microscopy (TEM) image by 3% phosphotungstate staining.

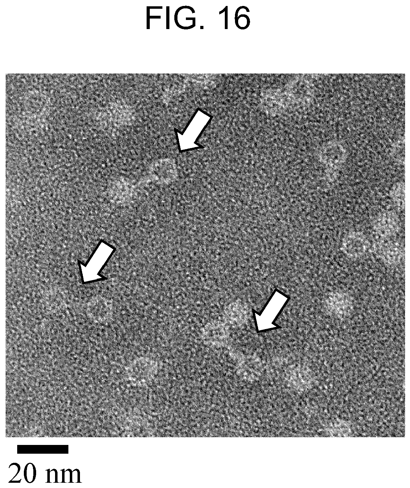

As a result, it was observed that two ferritin azide particles (cage structure at diameter: 12 nm) were linked via the antibodies (FIG. 16). Therefore, it was confirmed that the antibody-human ferritin complex obtained in Example 3 was an antibody linked to two human ferritin particles (the reactant of the azide group in the ferritin H chain in two human ferritin particles with two DBCO groups in the antibody).

### Example 5: Construction of antibody-human ferritin complex (2)

In this example, the DBCO modified antibody or DBCO-PEG modified antibody obtained in Example 1 and the ferritin azide particles obtained in Example 2 were reacted in several quantity ratio.

To PBS buffer at pH 7.4 in which the ferritin azide particles obtained in Example 2 were dissolved so as to be a final concentration of 1.0 µM, the DBCO modified antibody or DBCO-PEG modified antibody obtained in Example 1 was added so as to be a final concentration of 2.5 µM, 5.0 µM, or 12.5 µM, and the mixture was shaken at 25°C for 1 hour. At this time, the quantity ratio between the azide groups presented on the ferritin azide particles and the DBCO groups of the modified particles was 10:1, 5:1, or 2:1. The samples after reaction were diluted 5 times with water, mixed at 1:1 with Laemmli sample buffer (Biorad) containing 5% β-mercaptoethanol, and then heat-treated at 95°C for 5 minutes. A 10 µl aliquot of the solutions was analyzed by SDS-PAGE.

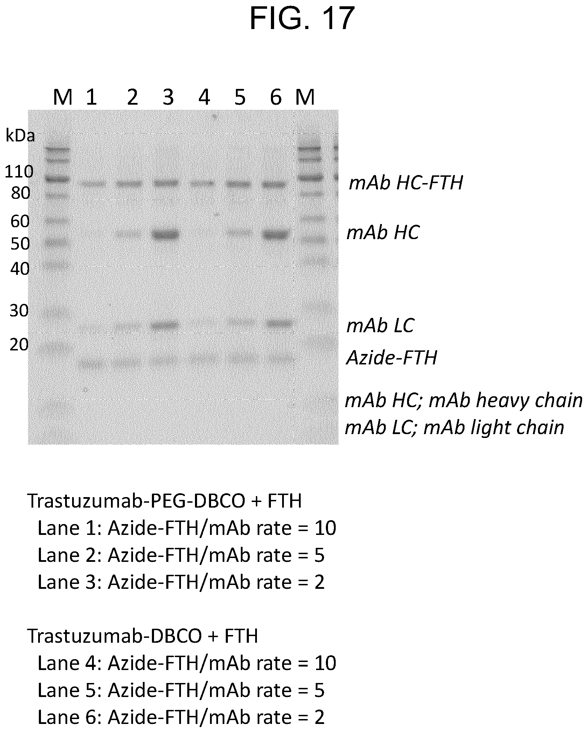

As a result, in all of the conjugation reaction samples, a protein band around 100 kDa where two ferritin H chains and an antibody heavy chain(s) were conjugatged was observed. In addition, when the ratio between the azide group in the ferritin azide particles and the DBCO in the antibody was 10:1, all of the antibody heavy chains were conjugated with the ferritin H chains (FIG. 17).

### Example 6: Construction of antibody-human ferritin complex (mosaic complex) (3)

In this example, in the antibody-human ferritin complex obtained in Example 5, the number of unreacted human ferritin azide H chains (human ferritin H chains introduced with bioorthogonal functional groups) was reduced.

The antibody-human ferritin complex obtained in Example 5 was suspended in 100 mM glycine hydrochloride buffer (pH 2.3) so that its concentration per ferritin reached a final concentration of 0.8 µM. To the solution, unmodified human ferritin particles (24-mer of unmodified human ferritin H chain) were added so as to be a final concentration of 20 µM, and the mixture was left at 25°C for 15 minutes to dissociate the cage structure of the ferritin particles. The human ferritin H chain linked to the antibody was then reassociated with the unmodified ferritin H chains by adding Tris-HCl buffer (pH 9.0) so as to be a final concentration of 200 mM.

As a result, by the reassociation, an antibody-human ferritin complex (mosaic complex) comprising a mosaic ferritin particle (a ferritin particle comprising a modified ferritin H chain and an unmodified ferritin H chain) characterized by comprising, as a 24-mer component, (a) a modified human ferritin H chain which is linked to the antibody by reacting the azide group with the DBCO group, (b) a human ferritin azide H chain which is not linked to the antibody (a modified human ferritin H chain introduced with the azide group that is a bioorthogonal functional group), and (c) an unmodified human ferritin H chain.

Next, the mosaic complexes were injected into Superdex200 increase 10/30 GL column (Cytiva) equilibrated with PBS (pH 7.4) and separated and purified by size at flow rate of 0.8 ml/min.

### Example 7: ESI-TOFMS analysis of antibody-human ferritin complex (mosaic complex)

A solution containing the antibody-human ferritin complex obtained in Example 6 was replaced with 20 mM ammonium acetate buffer, and the mass was measured by ESI-TOFMS according to the procedures previously reported (Anal. Chem., 2019, 91, 20, 12724-12732).

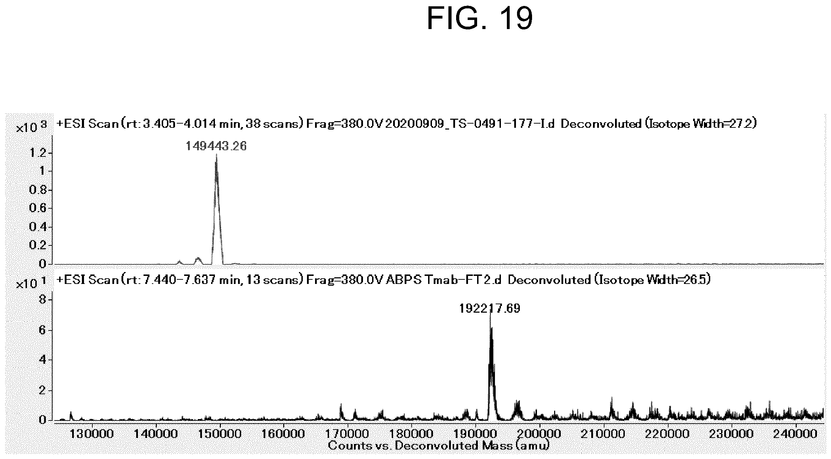

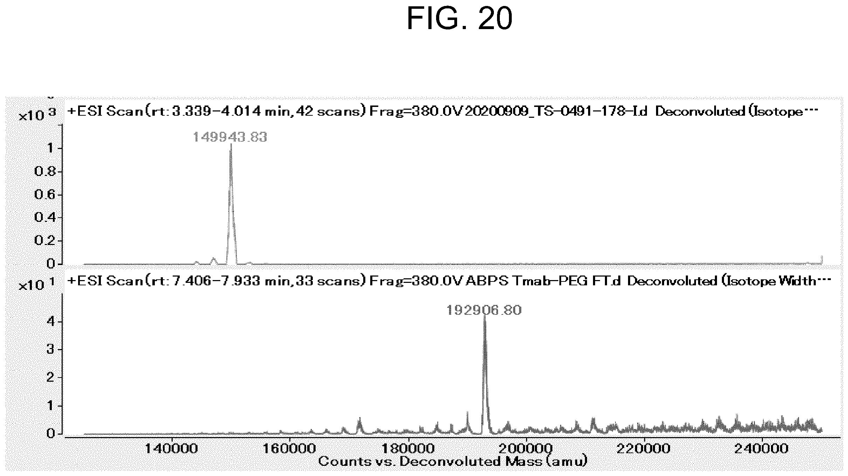

As a result, a peak at 192218 was confirmed as a complex in which two human ferritin H chains were introduced into the DBCO modified antibody synthesized in Example (1-1) (FIG. 19). Similarly, a peak at 192906 was confirmed as a complex in which two human ferritin H chains were introduced into the DBCO-PEG-modified antibody synthesized in Example (1-2) (FIG. 20).

### Example 8: Construction of mosaic ferritin particles

With 2 mL of 100 mM glycine hydrochloride buffer at pH 2.2, the unmodified human ferritin particles consisting of 24-mer of the unmodified human ferritin H chain and the ferritin azide particles obtained in Example 2 (modified human ferritin particles consisting of 24-mer of the modified human ferritin H chain introduced with the bioorthogonal functional group) were mixed so as to be final concentrations of 6.0 uM and 2.0 µM, respectively, and the mixture was left at room temperature for 15 minutes (dissociation process). Then, 0.2 mL of 1 M Tris-HCl buffer was added and the mixture was neutralized and allowed to stand at room temperature for 1 hour (reassociation process). By the dissociation and reassociation, mosaic ferritin particles are produced since the ferritin azide H chain can be replaced with an unmodified human ferritin H chain in the ferritin azide particles obtained in Example 2.

The resulting solution was injected into HiTrap Q FF 1 mL column (Cytiva) equilibrated with 50 mM Tris-HCl buffer (pH 8.0), and the mosaic ferritin particles were separated and purified by differences in surface charge at elution rate of 1 mL/min by utilizing salt concentration gradient with 50 mM Tris-HCl buffer (pH 8.0) containing 0 M to 1 M NaCl. The solution containing mosaic ferritin particles was replaced by 10 mM Tris-HCl buffer (pH 8.0) by centrifugal ultrafiltration using Vivaspn 20-100K (Cytiva).

Next, the ratio of the unmodified ferritin H chain and the modified ferritin H chain (ferritin azide H chain) constituting the recovered mosaic ferritin particles was analyzed by reverse phase chromatography. A 0.1% by volume aqueous trifluoroacetic acid solution was used as the mobile phase A, and an 80% by volume aqueous acetonitrile solution containing 0.1% by volume trifluoroacetic acid was used as the mobile phase B. 10 uL of samples were subjected to PLRP-S 300A 3 uM 150 × 4.6 MM (Agilent Technologies) equilibrated with a solution of 70% mobile phase A and 30% mobile phase B. The protein was then eluted by a linear gradient over 14 minutes so that the concentration of mobile phase B was from 30% to 75%. Column temperature was 70°C, flow rate was 1 mL/min, and detection was performed on absorbance at 220 nm.

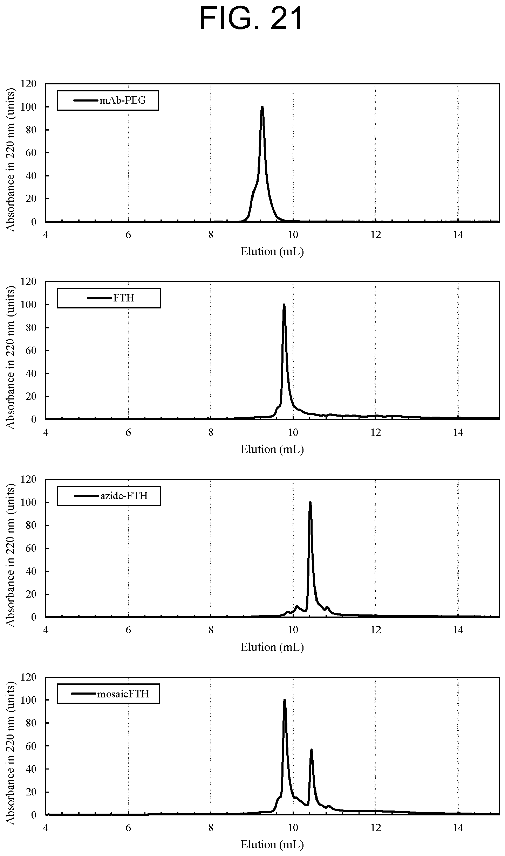

As a result, the unmodified ferritin H chain and the modified ferritin H chain (ferritin azide H chain) could be separated (FIG. 21). From the area ratio of the peaks obtained by analyzing the mosaic ferritin particles, it was found that the ratio of the modified ferritin H chain to the modified ferritin H chain (ferritin azide H chain) contained in the mosaic ferritin particles was 2.7 to 1.0, and that the average number of ferritin azide subunits was 6 to 7 per mosaic ferritin particle. It was found that the mosaic ferritin particles had an average of 12 to 14 azide groups per particle.

### Example 9: Construction of antibody-human ferritin Complex (mosaic complex) (4)

To a 50 mM HEPES buffer at pH 8.0 or 50 mM CHES buffer at pH 9.0 in which the mosaic ferritin particles obtained in Example 8 were dissolved so as to be a final concentration of 5.0 µM, the DBCO-PEG modified antibody obtained in Example 1 was added so as to be a final concentration of 1.0 µM, and the mixture was subjected to a shaking reaction at 25°C for 18 hours to generate an IgG antibody linked to two human ferritin particles (mosaic complex).

The resulting solutions were injected into HiTrap Q FF 1 mL column (Cytiva) equilibrated with 50 mM Tris-HCl buffer (pH 8.0), and the target protein was separated and purified by differences in surface charge at elution rate of 1 mL/min by utilizing salt concentration gradient with 50 mM Tris-HCl buffer (pH 8.0) containing 0 M to 0.5 M NaCl.

Next, the ratio of the unmodified ferritin H chain and the modified ferritin H chain (ferritin azide H chain) constituting the mosaic ferritin particles in the recovered mosaic complex was analyzed by reverse phase chromatography. A 0.1% by volume aqueous trifluoroacetic acid solution was used as the mobile phase A, and a 80% by volume aqueous acetonitrile solution containing 0.1% by volume trifluoroacetic acid was used as the mobile phase B. 10 uL of samples were subjectged to PLRP-S 300A 3 uM 150 × 4.6 MM (Agilent Technologies) equilibrated with a solutuon of 70% mobile phase A and 30% mobile phase B. The protein was then eluted by a linear gradient over 14 minutes so that the concentration of mobile phase B was from 30% to 75%. Column temperature was 70°C, flow rate was 1 mL/, and and detection was performed on absorbance at 220 nm.

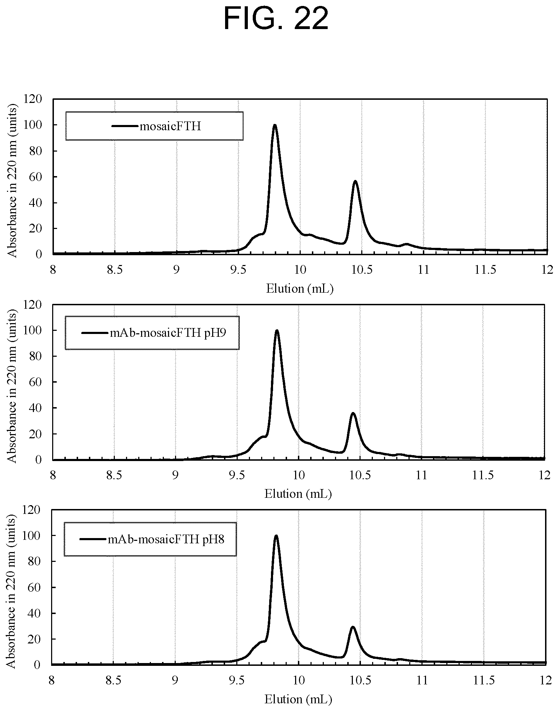

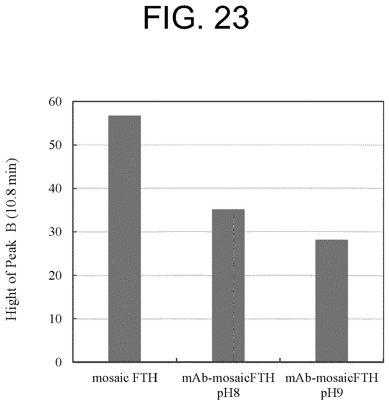

As a result, it was found that the ratio between the peak of the unmodified ferritin H chain and the peak of the modified ferritin H chain (ferritin azide H chain) was changed by the antibody modification reaction, and the peak of the modified ferritin H chain (ferritin azide H chain) was decreased to 50 to 60% before the antibody modification reaction (FIG. 22 and FIG. 23). In this analysis condition, the elution position of each human ferritin H chain is as follows.
(a) Unmodified ferritin H chain: 9.8 min
(b) Modified ferritin H chain which is not linked to an antibody (ferritin azide H chain): 10.4 min
(c) Modified ferritin H chain which is linked to an antibody: 9.8 min

In this analysis condition, the elution position of the modified ferritin H chain which is linked to the antibody is equivalent to the elution position of the unmodified ferritin H chain, and the increase or decrease of the modified ferritin H chain which is linked to the antibody cannot be directly confirmed. Therefore, it was considered that the decrease in the peak of the modified ferritin H chain (ferritin azide H chain) observed this time was caused by the decrease in the modified ferritin H chain (ferritin azide H chain) due to the production of the modified ferritin H chain which is linked to the antibody by the reaction between the modified ferritin H chain (ferritin azide H chain) and the antibody.

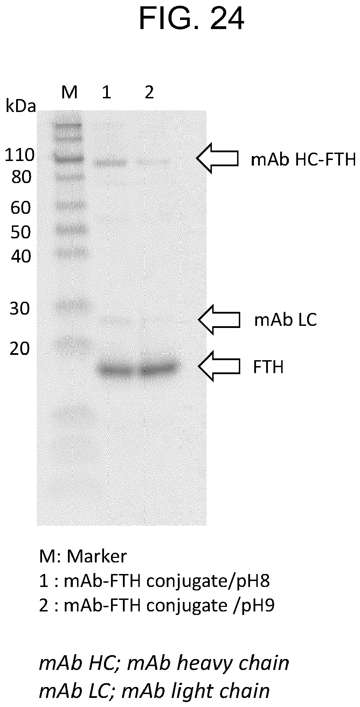

The obtained sample was mixed at 1:1 with Laemmli sample buffer (Biorad) containing 5% β-mercaptoethanol, and then heat-treated at 95°C for 5 minutes. A 10 µl aliquot of the solution was analyzed by SDS-PAGE. As a result, a protein band around 100 kDa in which the ferritin H chain and the antibody heavy chain were conjugated was observed only in the conjugated reaction sample (FIG. 24), which was not observed by the antibody or the ferritin particle alone. The molecular weight of the ferritin azide H chain (monomer) is 21 kDa and the molecular weight of the antibody heavy chain is from 50 kDa to 60 kDa. Therefore, it was considered that the band obtained by the conjugation reaction was a protein in which two ferritin azide H chains (monomer) were conjugated to the antibody heavy chain via covalent bonds.

[Sequence Listing]

## Claims

1. A complex or salt thereof comprising (A) one IgG antibody and (B) two human ferritin particles comprising one or more human ferritin H chains linked thereto.

2. The complex or salt thereof according to claim 1, wherein the human ferritin particle is a 24-mer of the human ferritin H chain.

3. The complex or salt thereof according to claim 1 or 2, wherein the human ferritin H chain is a natural-type human ferritin H chain which may lack a methionine residue at position 1.

4. The complex or salt thereof according to any one of claims 1 to 3, wherein the complex is represented by the following formula (1):
X1-L1-Y-L2-X2 (1)
wherein
X1 and X2 each indicate a human ferritin particle comprising one or more human ferritin H chains,
Y is an IgG antibody,
L1 and L2 each indicate a linker, and
the two human ferritin particles indicated by X1 and X2 are linked to two linkers indicated by L1 and L2, respectively, via an atom in the side chain of amino acid residues present at the same position in the human ferritin H chain contained therein.

5. The complex or salt thereof according to claim 4, wherein the linker does not contain a peptide moiety.

6. The complex or salt thereof according to claim 4 or 5, wherein the atom in the side chain of the amino acid residue present at the same position in the human ferritin H chain is the sulfur atom in the side chain of cysteine residue.

7. The complex or salt thereof according to claim 6, wherein the cysteine residue is present at position 91 and/or position 103 according to the reference position of the natural-type human ferritin H chain.

8. The complex or salt thereof according to any one of claims 1 to 7, wherein the antibody is a chimeric monoclonal antibody, a humanized monoclonal antibody, or a human monoclonal antibody.

9. A complex or salt thereof according to any one of claims 4 to 8, wherein the IgG antibody indicated by Y is linked to two linkers indicated by L1 and L2 via an atom in the side chain of amino acid residues present at the same position in the two heavy chain constant regions.

10. The complex or salt thereof according to claim 9, wherein the atom in the side chain of the amino acid residues present at the same position in the two heavy chain constant regions is the nitrogen atom in the side chain of lysine residue.

11. The complex or salt thereof according to claim 10, wherein the lysine residue is present at position 246/248, 288/290, or 317 of the IgG antibody heavy chain according to EU numbering.

12. The complex or salt thereof according to any one of claims 1 to 11, wherein the human ferritin particles comprising one or more human ferritin H chains comprise one or more modified human ferritin H chains and one or more unmodified human ferritin H chains.

13. The complex or salt thereof according to any one of claims 1 to 12, wherein the human ferritin particle is a particle comprising, per particle, each human ferritin H chain of the following (a) to (c) in the following average number:
(a) the number of the modified human ferritin H chains linked to the linker is 1;
(b) the number of the modified human ferritin H chains which are not linked to the linker and which are introduced with a modifying group is n; and
(c) the number of the unmodified human ferritin H chains is m;
where n is a number from 0 to 23, m is a number from 0 to 23, and the sum of n and m is 23.

14. The complex or salt thereof according to any one of claims 1 to 13, wherein the human ferritin particles are particles comprising one or more unmodified human ferritin H chains on average.

15. The complex or salt thereof according to any one of claims 1 to 14, wherein the human ferritin particles are particles comprising 12 or more unmodified human ferritin H chains on average.

16. The complex or salt thereof according to any one of claims 13 to 15, wherein the modifying group comprises a reactive group.

17. The complex or salt thereof according to claim 16, wherein the reactive group is a bioorthogonal functional group to a protein.

18. The complex or salt thereof according to claim 16, wherein the bioorthogonal functional group to the protein comprises one or more partial structures selected from the group consisting of a maleimide moiety, an azide moiety, a ketone moiety, an aldehyde moiety, a thiol moiety, an alkene moiety, an alkyne moiety, a halogen moiety, a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety, an isonitrile moiety, a sydnone moiety, and a selenium moiety.

19. The complex or salt thereof according to any one of claims 13 to 15, wherein the modifying group comprises a functional substance.

20. The complex or salt thereof according to claim 19, wherein the functional substance comprises one or more moieties selected from the group consisting of a peptide, a protein, a nucleic acid, a small organic compound, a chelator, a sugar chain, a lipid, a high polymer, and a metal.

21. The complex or salt thereof according to any one of claims 1 to 20, wherein the human ferritin particles have a substance in an internal cavity.

22. A method for producing a complex or salt thereof,
wherein the complex comprises (A) one IgG antibody and (B) two human ferritin particles comprising one or more human ferritin H chains linked thereto,
wherein the method comprises the following (1)-(3):
(1) introducing first reactive groups into an IgG antibody to form a modified IgG antibody comprising two first reactive groups;
(2) introducing a second reactive group capable of reacting with the first reactive group into human ferritin particles comprising one or more human ferritin H chains to form modified particles comprising one or more modified human ferritin H chains comprising the second reactive group; and
(3) reacting the modified IgG antibody with the modified particles to obtain the complex.

23. The method according to claim 22, wherein the complex is represented by the following formula (1):
X1-L1-Y-L2-X2 (1)
wherein
X1 and X2 each indicate a human ferritin particle comprising one or more human ferritin H chains,
Y is an IgG antibody,
L1 and L2 each indicate a linker, and
the two human ferritin particles indicated by X1 and X2 are linked to two linkers indicated by L1 and L2, respectively, via an atom in the side chain of amino acid residues present at the same position in the human ferritin H chain contained therein.

24. The method according to claim 23, wherein the linker does not contain a peptide moiety.

25. The method according to claim 23 or 24, wherein the atom in the side chain of the amino acid residue present at the same position in the human ferritin H chain is the sulfur atom in the side chain of cysteine residue.

26. The method according to claim 25, wherein the cysteine residue is present at position 91 and/or 103 according to the reference position of a natural-type human ferritin H chain.

27. A method for producing a complex or salt thereof,
wherein the complex comprises (A) one IgG antibody and (B) two human ferritin particles comprising one or more modified human ferritin H chains and one or more unmodified human ferritin H chains linked thereto,
wherein the method comprises the following (1)-(4):
(1) introducing first reactive groups into an IgG antibody to form a modified IgG antibody comprising two first reactive groups;
(2) introducing a second reactive group capable of reacting with the first reactive group into human ferritin particles comprising two or more human ferritin H chains to form first modified particles comprising two or more modified human ferritin H chains comprising the second reactive group;
(3) replacing a portion of the two or more modified human ferritin H chains in the first modified particles with unmodified human ferritin H chains to form second modified particles comprising one or more modified human ferritin H chains and one or more unmodified human ferritin H chains; and
(4) reacting the modified IgG antibody with the second modified particles to obtain the complex.

28. A method for producing a complex or salt thereof,
wherein the complex comprises (A) one IgG antibody and (B) two human ferritin particles comprising one or more modified human ferritin H chains and one or more unmodified human ferritin H chains linked thereto,
wherein the method comprises the following (1)-(4):
(1) introducing first reactive groups into an IgG antibody to form an IgG antibody comprising two first reactive groups;
(2) introducing a second reactive group capable of reacting with the first reactive group into human ferritin particles comprising two or more human ferritin H chains to form first modified particles comprising two or more modified human ferritin H chains comprising the second reactive group;
(3) reacting the modified IgG antibody with the first modified particles to form a complex or salt thereof comprising (A') one IgG antibody and (B') two human ferritin particles comprising two or more modified human ferritin H chains linked thereto; and
(4) replacing a portion of the two or more modified human ferritin H chains in the complex or salt thereof comprising (A') one IgG antibody and (B') two human ferritin particles comprising two or more modified human ferritin H chains linked thereto with an unmodified human ferritin H chain to obtain the complex or salt thereof comprising (A) one IgG antibody and (B) two human ferritin particles comprising one or more modified human ferritin H chains and one or more unmodified human ferritin H chains linked thereto.

29. The method according to claim 27 or 28, wherein the complex is represented by the following formula (1):
X1-L1-Y-L2-X2 (1)
wherein
X1 and X2 each indicate a human ferritin particle comprising one or more modified human ferritin H chains and one or more unmodified human ferritin H chains,
Y is an IgG antibody,
L1 and L2 each indicate a linker, and
the two human ferritin particles indicated by X1 and X2 are linked to two linkers indicated by L1 and L2, respectively, via an atom in the side chain of amino acid residues present at the same position in the human ferritin H chain contained therein.

30. The method according to claim 29, wherein the linker does not contain a peptide moiety.

31. The method according to claim 30, wherein the atom in the side chain of the amino acid residues present at the same position in the human ferritin H chain is the sulfur atom in the side chain of cysteine residue.

32. The method according to claim 31, wherein the cysteine residue is present at position 91 and/or 103 according to the reference position of the natural-type human ferritin H chain.

33. The method according to any one of claims 22 to 32, wherein the first reactive group and the second reactive group are a bioorthogonal functional group to a protein.

34. The method according to claim 33,
wherein the bioorthogonal functional group to the protein comprises one or more partial structures selected from the group consisting of a maleimide moiety, an azide moiety, a ketone moiety, an aldehyde moiety, a thiol moiety, an alkene moiety, an alkyne moiety, a halogen moiety, a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety, an isonitrile moiety, a sydnone moiety, and a selenium moiety,
wherein the first reactive group and the second reactive group are selected in a combination capable of reacting with each other.
